# EUROPEAN PATENT SPECIFICATION

(11) **EP 3 698 711 B1**
(45) Date of publication and mention of the grant of the patent: **03.12.2025**
(21) Application number: 18868171.2
(22) Date of filing: 19.10.2018
(51) Int. Cl.: A61B 5/369, A61H 1/02, A61B 5/00

(54) **ELECTROENCEPHALOGRAM MEASUREMENT SYSTEM. REHABILITATION SUPPORT SYSTEM, METHOD FOR CONTROLLING THE ELECTROENCEPHALOGRAM MEASUREMENT SYSTEM, PROGRAM, AND NON-TRANSITORYSTORAGE MEDIUM**
SYSTEM ZUR ELEKTROENZEPHALOGRAMM MESSUNG, REHABILITATIONSUNTERSTÜTZUNGSSYSTEM, VERFAHREN ZUR STEUERUNG EINES ELEKTROENZEPHALOGRAMM MESSSYSTEMS, PROGRAMM UND NICHTTRANSITORISCHES AUFZEICHNUNGSMEDIUM
SYSTÈME DE MESURE D'ÉLECTROENCÉPHALOGRAMME, SYSTÈME DE SUPPORT DE RÉÉDUCATION, PROCÉDÉ DE COMMANDE DE SYSTÈME DE MESURE D'ÉLECTROENCÉPHALOGRAMME, PROGRAMME ET SUPPORT D'ENREGISTREMENT NON-TRANSITOIRE

(30) Priority: 20.10.2017 JP 2017204087
(43) Date of publication of application: 26.08.2020
(73) Proprietor: LIFESCAPES Inc., Tokyo (JP)
(72) Inventor: HIRATA, Akio, Osaka 540-6207 (JP); MATSUMOTO, Akinori, Osaka 540-6207 (JP); MORIKAWA, Koji, Osaka 540-6207 (JP)
(74) Representative: Appelt, Christian W.
(86) International application number: PCT/JP2018/038935
(87) International publication number: WO 2019/078330

(56) References cited:
- WO-A1-2013/038285
- WO-A2-2012/034014
- CN-A- 103 142 225
- JP-A- 2003 038 454
- JP-A- 2012 217 721
- JP-A- 2013 066 526
- JP-A- 2016 013 180
- JP-A- 2016 013 182
- US-A1- 2006 020 218
- US-A1- 2011 130 675
- US-A1- 2015 257 674
- KATO, ZOJIRO ET AL.: "Passage, [How to Measure Biological Function for Beginners]", [HOW TO MEASURE BIOLOGICAL FUNCTION FOR BEGINNERS] SECOND EDITION, 2006, TOKYO, pages 33 - 35, XP009520425, ISBN: 4-88922-104-2
- SUENAGA, KAZUE ET AL.: "Passage, Latest EEG standard text", LATEST EEG STANDARD TEXT, 5TH EDITION, 2016, TOKYO, pages 218 - 220, XP009520423

## Description

### Technical Field

The present disclosure generally relates to an electroencephalogram (EEG) measurement system, a rehabilitation support system, a method for controlling the electroencephalogram measurement system, a program, and a non-transitory storage medium. More particularly, the present disclosure relates to an electroencephalogram measurement system, a rehabilitation support system, a method for controlling the electroencephalogram measurement system, a program, and a non-transitory storage medium, all of which are configured or designed to acquire electroencephalogram information representing a subject-specific electroencephalogram obtained by an electrode unit that is placed on a region of interest (ROI) forming part of a subject's head.

### Background Art

Various techniques for measuring a subject's electroencephalogram and assisting, based on the electroencephalogram measured, him or her in exercising a region belonging to his or her half body have been proposed.

For example, JP 2016-129660 A discloses a rehabilitation support system (rehabilitation system), which includes an electrode unit (head electrode) and an exercise assisting device (rehabilitation assisting device) equipped with an actuator. The electrode unit is placed on a region of the subject's (or user's) head (corresponding to a region of interest) which is used by him or her to exercise his or her fingers. The electrode unit includes electrodes for measuring an electroencephalogram produced responsive to the subject's movement intention. The exercise assisting device is worn by the subject on his or her hand to assist him or her in the action of stretching his or her fingers. This rehabilitation support system drives, when the electrode unit measures an electroencephalogram representing the subject's movement intention while the subject is made to imagine stretching his or her fingers, the actuator of the exercise assisting device, thereby assisting the subject in the action of opening his or her hand.

In the rehabilitation support system of JP 2016-129660 A, while the subject is exercising his or her fingers, the electrode unit may be displaced due to his or her body movement, for example. If the displacement of the electrode unit causes an increase in impedance value between the electrode unit and the subject's body, the measurement accuracy of the electroencephalogram information (electroencephalogram) may deteriorate.

US 2015/257674 A1 discloses a device for performing EEG which consists of an elastic head strap, electrodes, and a wireless transmitter.

US 2011/130675 A1 discloses a quantitative electroencephalogram (QEEG) monitor and system for monitoring and displaying simultaneously neuropathological characteristic and activity of both sides of a subject's brain.

WO 2012/034014 A2 discloses an apparatus comprising a headset and a control unit in communication with each other.

WO 2013/038285 A1 discloses an EEG system comprising a first EEG electrode, providing a first EEG signal and a first impedance signal, and a second EEG electrode, providing a second EEG signal and a second impedance signal.

JP 2016-013182 A discloses another rehabilitation support system.

### Summary of Invention

It is therefore an object of the present disclosure to provide a rehabilitation support system and a method for controlling the system, all of which are configured or designed to improve the measurement accuracy of electroencephalogram information.

A rehabilitation support system and a method for controlling the rehabilitation support system according to the present invention are defined in independent claims 1 and 11, respectively. Preferred embodiments are defined in dependent claims.

### Brief Description of Drawings

FIG. 1 schematically illustrates how an electroencephalogram measurement system according to an exemplary embodiment and a rehabilitation support system including the electroencephalogram measurement system may be used;
FIG. 2 is a schematic front view illustrating how the headset of the electroencephalogram measurement system may be used;
FIG. 3 is a block diagram illustrating a configuration for the electroencephalogram measurement system and the rehabilitation support system;
FIG. 4 is a circuit diagram of a main section of the electroencephalogram measurement system;
FIG. 5 illustrates an exemplary wearing condition check screen of the electroencephalogram measurement system;
FIG. 6 is a flowchart showing the procedure of decision processing to be performed by the electroencephalogram measurement system; and
FIG. 7 illustrates an exemplary training screen of the electroencephalogram measurement system.

### Description of Embodiments

### (First embodiment)

### (1) Overview

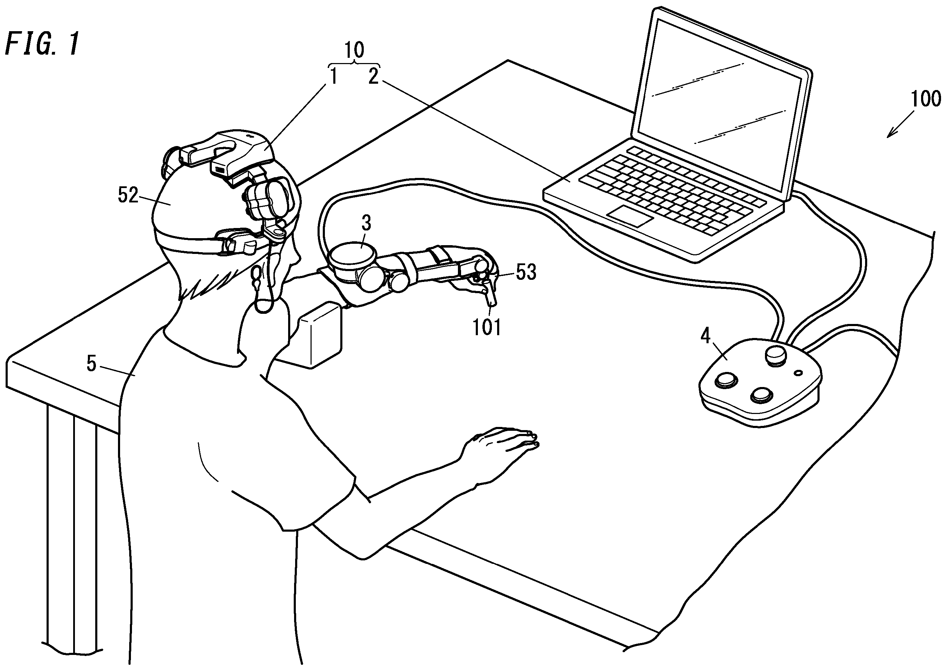

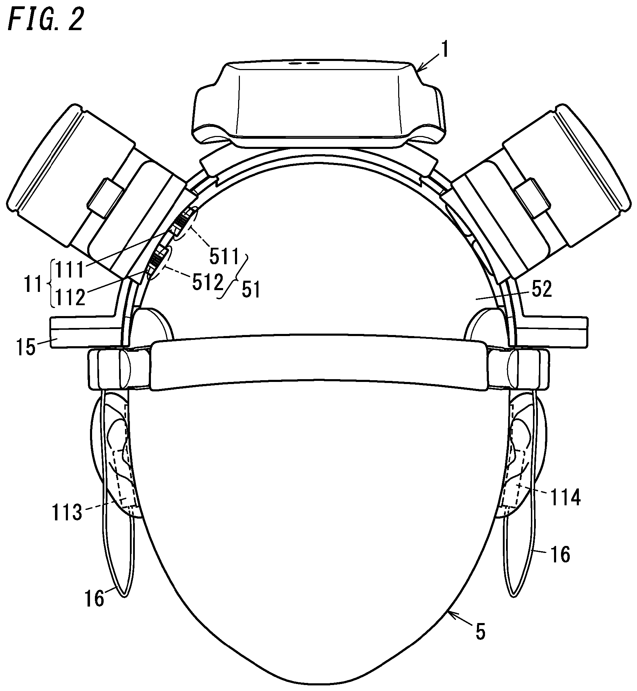

An overview of an electroencephalogram measurement system 10 according to an exemplary embodiment will be described with reference to FIGS. 1 and 2.

The electroencephalogram measurement system 10 according to this embodiment is a system for measuring an electroencephalogram specific to a subject 5, and acquires electroencephalogram information representing a subject-specific electroencephalogram obtained by an electrode unit 11 placed on a region of interest 51 that forms part of the subject's 5 head 52. As used herein, the "electroencephalogram (EEG)" refers to a waveform recorded by deriving, out of a human's body, electrical signals (action potentials) generated by (groups of) nerve cells (or neurons) of a human brain. Also, as used herein, an "electroencephalogram" refers to, unless otherwise stated, an on-scalp electroencephalogram obtained by recording, using the electrode unit 11 worn by the subject 5 on his or her body surface, a comprehensive action potential of a great many groups of neurons of the cerebral cortex.

The electroencephalogram measurement system 10 includes a headset 1 with the electrode unit 11 and an information processor 2. The headset 1 is worn by a subject 5 on his or her head 52 with the electrode unit 11 brought into contact with his or her head 52 surface (i.e., scalp). According to the present disclosure, the electrode unit 11 is mounted on paste (electrode paste) applied onto the surface of the head 52, and thereby comes into contact with the surface of the head 52. In this case, the electrode unit 11 comes into direct contact with (i.e., not via the subject's hairs) the surface of the head 52 by pushing the hairs aside. Naturally, the electrode unit 11 may come into direct contact with the surface of the head 52 with no paste applied between them. That is to say, according to the present disclosure, "to bring the electrode unit 11 into contact with the surface of the head 52" refers to not only bringing the electrode unit 11 into direct contact with the surface of the head 52 but also bringing the electrode unit 11 into indirect contact with the surface of the head 52 with some intermediate interposed between the electrode unit 11 and the surface of the head 52. The intermediate does not have to be paste but may also be a gel with electrical conductivity.

The headset 1 measures the electroencephalogram specific to the subject 5 by having the electrode unit 11 measure the action potential of the subject's 5 brain, thereby generating electroencephalogram information representing the electroencephalogram. The headset 1 may transmit the electroencephalogram information to the information processor 2 by wireless communication, for example.

In response, the information processor 2 subjects the electroencephalogram information acquired from the headset 1 to various types of processing or displays the electroencephalogram information thereon.

In the following description of embodiments, a situation where the electroencephalogram measurement system 10 is used in a rehabilitation support system 100 for supporting the subject 5 in his or her rehabilitation will be described. That is to say, the rehabilitation support system 100 includes the electroencephalogram measurement system 10 according to this embodiment. The rehabilitation support system 100 further includes an exercise assisting device 3 and a controller 4. The exercise assisting device 3 assists the subject 5 with his or her exercise by applying at least one of a mechanical stimulus or an electrical stimulus to the subject 5. The controller 4 controls the exercise assisting device 3 based on the electroencephalogram information acquired by the electroencephalogram measurement system 10.

This rehabilitation support system 100 supports a subject 5, who suffers from either a motor paralysis or a decline in motor function in some region of his or her body due to some brain disease such as cerebral apoplexy (stroke) or a traffic accident, in his or her rehabilitation by exercise therapy. Such a subject 5 may either be unable to do, or show a decline in the physical ability to do well, a voluntary movement, which is a movement that the subject 5 does of his or her own will or by intention. As used herein, the "exercise therapy" refers to a method for recovering a voluntary movement function for an affected region of the subject's 5 body by making the subject 5 exercise his or her region that is either unable to do, or shows a decline in the physical ability do well, such a voluntary movement (hereinafter referred to as "affected region").

In the following description of embodiments, the rehabilitation support system 100 is supposed to be used, for example, to support the subject 5 in his or her rehabilitation to recover the function of his or her left fingers 53. That is to say, in this case, the subject's 5 left fingers are his or her affected region. However, this is only an example and should not be construed as limiting. Alternatively, the rehabilitation support system 100 may also be used to support the subject 5 in his or her rehabilitation to recover the proper function of his or her right fingers.

The rehabilitation support system 100 supports the subject 5 in his or her voluntary movement by having the exercise assisting device 3, which the subject 5 wears on his or her left hand, apply at least one of a mechanical stimulus or an electrical stimulus to his or her left hand when the subject 5 does the voluntary movement using his or her left fingers 53. This allows, just like a situation where a medical staff such as a physical therapist or an occupational therapist supports the subject 5 in his or her voluntary movement by holding the subject's 5 fingers 53, the rehabilitation support system 100 to support him or her in the voluntary movement. Thus, the rehabilitation support system 100 is able to provide rehabilitation by exercise therapy more effectively than in a situation where the subject 5 does the voluntary movement by him- or herself, to say nothing of a situation where some medical staff provides the support.

Meanwhile, to provide support for such rehabilitation, the rehabilitation support system 100 suitably assists, using the exercise assisting device 3, the subject 5 with his or her voluntary movement when the subject 5 is going to do the voluntary movement on his or her own. The rehabilitation support system 100 assists, when the subject 5 is going to do voluntary movement, him or her in the voluntary movement using the exercise assisting device 3 by operating the exercise assisting device 3 in coordination with the subject's 5 electroencephalogram (electroencephalogram information) measured by the electroencephalogram measurement system 10. In other words, the rehabilitation support system 100 provides rehabilitation by exercise therapy by using the brain-machine interface (BMI) technology.

When the subject 5 is going to do voluntary movement (i.e., while the subject 5 is doing the voluntary movement), a characteristic variation may arise in his or her electroencephalogram. That is to say, when the subject 5 plans to do (or imagines doing) the voluntary movement, a brain region corresponding to the region that should be exercised to do the voluntary movement may be activated. Examples of such brain regions include a somatosensory motor cortex. Supporting the subject 5 in his or her voluntary movement using the exercise assisting device 3 at the timing when the brain region is activated would make the rehabilitation even more effective. Such brain region activation may be detected as a characteristic variation in electroencephalogram. Thus, the rehabilitation support system 100 starts supporting the subject 5 in his or her voluntary movement using the exercise assisting device 3 at the timing when this characteristic variation arises in the electroencephalogram specific to the subject 5. Note that such a characteristic variation may arise in the electroencephalogram even if the voluntary movement is not actually carried out but when the subject 5 imagines doing the voluntary movement (i.e., plans to do the movement). That is to say, this characteristic variation may arise in the electroencephalogram even if the voluntary movement is not actually carried out but when the subject 5 plans to do, or imagines doing, the voluntary movement to activate the corresponding brain region. Therefore, the rehabilitation support system 100 may also support even a subject 5, who is unable to do the voluntary movement, in his or her attempt to do the voluntary movement.

The rehabilitation support system 100 with such a configuration is able to provide effective rehabilitation by exercise therapy for the subject 5 while lightening the workload on medical staff. In addition, this rehabilitation support system 100 eliminates the variation in timing to start supporting the subject 5 in his or her voluntary movement due to a human factor such as the skill level of the medical staff who needs to support the subject 5 in his or her voluntary movement, thus reducing the variation in the effect of rehabilitation. In particular, the rehabilitation support system 100 is able to start supporting the subject 5 in his or her voluntary movement at the timing when a characteristic variation arises in his or her specific electroencephalogram (i.e., the timing when the brain region is actually activated). As can be seen, this rehabilitation support system 100 allows the subject 5 to start training at the timing when his or her brain activity starts, thus contributing to learning and establishing right brain activity. Among other things, it is difficult for even the subject 5 him- or herself and the medical staff to determine whether or not such a characteristic variation has arisen in his or her electroencephalogram. Thus, using this rehabilitation support system 100 provides highly effective rehabilitation that is usually difficult to realize by either the subject 5 or the medical staff alone.

In the embodiment to be described below, when the subject 5 uses the rehabilitation support system 100, the subject 5 is supposed to be accompanied by some medical staff such as a physical therapist or an occupational therapist and the rehabilitation support system 100 is supposed to be operated by the medical staff. However, the subject 5 who uses the rehabilitation support system 100 does not have to be accompanied by medical staff. Alternatively, the rehabilitation support system 100 may be operated by either the subject 5 him- or herself or his or her family member as well.

The electroencephalogram measurement system 10 according to this embodiment is used in the rehabilitation support system 100 in order to detect a target electroencephalogram, which is an electroencephalogram with a characteristic variation that arises when the subject 5 is going to do the voluntary movement (i.e., when the subject 5 plans to do the voluntary movement). As will be described in detail later in the "(2) Rehabilitation support system" section, the electroencephalogram measurement system 10 detects, as the characteristic variation, a variation caused due to event-related desynchronization (ERD) in the intensity of the electroencephalogram within a particular frequency band. As used herein, the "event-related desynchronization" refers to a decline in power falling within a particular frequency band of the electroencephalogram representing a brain wave measured in the vicinity of a motor area during the voluntary movement (or when the subject 5 just imagines doing the voluntary movement). As used herein, the phrase "during the voluntary movement" refers to a process that begins when the subject 5 plans to do (or imagines doing) the voluntary movement and ends when the voluntary movement is either done successfully or ends up in failure. The "event-related desynchronization" may be triggered, during the voluntary movement, by the subject's plan to do the voluntary movement (or his or her image of doing the voluntary movement). The frequency bands in which the event-related desynchronization causes a decline in power are mainly an α wave range (such as a frequency band from 8 Hz to less than 13 Hz) and a β wave range (such as a frequency band from 13 Hz to less than 30 Hz).

The electroencephalogram measurement system 10 according to this embodiment measures an electroencephalogram with the electrode unit 11 brought into contact with the surface of the subject's 5 head 52 (i.e., his or her scalp). Therefore, to measure the electroencephalogram accurately, the impedance value between the electrode unit 11 and the subject's 5 body (such as his or her head 52) needs to be reduced as much as possible. The impedance value between the electrode unit 11 and the subject's 5 body (such as his or her head 52) varies according to the condition of contact between the surface of the head 52 and the electrode unit 11. For example, if there is any foreign matter such as hairs between the surface of the head 52 and the electrode unit 11, the impedance value between the electrode unit 11 and the subject's 5 body increases. Also, in a situation where the subject 5 performs training of his or her affected region for a long time (e.g., for one hour), displacement of the electrode unit 11 due to his or her body movement during the training, for example, may cause an increase in impedance value between the electrode unit 11 and the body of the subject 5.

An electroencephalogram measurement system 10 according to this embodiment includes an acquisition unit 211, an impedance measurement unit 121, and an output unit 212. The acquisition unit 211 acquires electroencephalogram information representing an electroencephalogram obtained by an electrode unit 11 that is placed on a region of interest 51 forming part of a subject's 5 head 52. The impedance measurement unit 121 measures an impedance value between the electrode unit 11 and the subject's 5 body in an acquisition mode in which the acquisition unit 211 acquires the electroencephalogram information. The output unit 212 outputs the impedance value measured. The impedance value measured may be output as, for example, a message displayed, a verbal message emitted, a page printed out, a file written into a non-transitory storage medium, or data transmitted to a telecommunications device. This allows the user to determine, based on the impedance value measured as provided by the output unit 212, the condition of contact between the electrode unit 11 and the subject's 5 head 52. Thus, if the condition of contact between the electrode unit 11 and the subject's 5 head 52 is not good, then the user may rectify the arrangement of the electrode unit 11, thus improving the measurement accuracy of electroencephalogram information.

### (2) Rehabilitation support system

Next, a rehabilitation support system 100 according to this embodiment will be described in further detail.

As shown in FIG. 1, the rehabilitation support system 100 includes the electroencephalogram measurement system 10, the exercise assisting device 3, and the controller 4.

As described above, the electroencephalogram measurement system 10 according to this embodiment includes the headset 1 and the information processor 2.

As shown in FIG. 2, the headset 1 is worn by the subject 5 on his or her head 52. The headset 1 includes the electrode unit 11. The electrode unit 11 is placed on a region of interest 51, which forms part of the subject's 5 head 52. Specifically, the headset 1 has the subject's 5 electroencephalogram measured by the electrode unit 11 that is brought into contact with the region of interest 51 defined on an area of the surface (scalp) of the subject's 5 head 52, thereby generating electroencephalogram information representing the electroencephalogram.

The information processor 2 includes, as its main constituent element, a computer system such as a personal computer. The information processor 2 receives the electroencephalogram information from the headset 1 via wireless communication and performs various types of processing on the electroencephalogram information. In this embodiment, detection of an electroencephalogram with a characteristic variation that may arise when the subject 5 plans to do (or imagines doing) the voluntary movement, the calibration processing, and other types of processing are performed by the information processor 2.

When the subject 5 plans to do (or imagines doing) the voluntary movement, the electroencephalogram measured usually comes to have a characteristic variation that represents a brain wave produced in the motor area corresponding to a body region where the voluntary movement is conducted. Thus, the electroencephalogram measurement system 10 regards, as the target of measurement, the electroencephalogram detected from around the motor area corresponding to the affected region as the target of rehabilitation. In this case, the motor area corresponding to left fingers is located on the right side of the brain and the motor area corresponding to right fingers is located on the left side of the brain. That is why when the subject's 5 left fingers 53 are the target of rehabilitation as in this embodiment, the electroencephalogram obtained by the electrode unit 11 that is brought into contact with the right side of the subject's 5 head 52 is the target of measurement for this electroencephalogram measurement system 10. That is to say, the electrode unit 11 is placed on a region of interest 51 that forms part of the right surface of the subject's 5 head 52 as shown in FIG. 2. For example, the electrode unit 11 is placed at a location designated by the mark "C4" according to the international 10-20 system. On the other hand, when the subject's 5 right fingers are the target of rehabilitation, the electrode unit 11 is placed on a region of interest that forms part of the left surface of the subject's 5 head 52. For example, the electrode unit 11 may be placed at a location designated by the mark "C3" according to the international 10-20 system in that case.

On detecting an electroencephalogram with a characteristic variation that may arise when the subject 5 plans to do voluntary movement, the electroencephalogram measurement system 10 outputs a control signal for controlling the exercise assisting device 3. That is to say, in this rehabilitation support system 100, generation of a control signal for controlling the exercise assisting device 3 is triggered by detection by the electroencephalogram measurement system 10 of an electroencephalogram with a characteristic variation that may arise when the subject 5 plans to do voluntary movement. Thus, this rehabilitation support system 100 allows the exercise assisting device 3 to assist the subject 5 with his or her voluntary movement when the subject 5 is going to do the voluntary movement. The electroencephalogram measurement system 10 will be described in further detail later in the "(3) Electroencephalogram measurement system" section.

The exercise assisting device 3 is a device for assisting the subject 5 with his or her exercise by applying at least one of a mechanical stimulus or an electrical stimulus to the subject 5. In this embodiment, the rehabilitation support system 100 is used to support the subject 5 in his or her rehabilitation to recover the function of his or her left fingers, and therefore, the exercise assisting device 3 is worn by the subject 5 on his or her left hand as shown in FIG. 1.

In the following description of this embodiment, the rehabilitation support system 100 is supposed to be used, for example, to support the subject 5 in his or her rehabilitation to recover the function of gripping something with his or her left fingers and the function of stretching his or her left fingers. As used herein, the "gripping action" refers to the action of gripping something. Also, as used herein, the "stretching action" refers to the action of opening a hand by stretching four fingers 53 (namely, second to fifth fingers), except the first finger (i.e., the thumb), or the action of releasing an "object" that the subject 5 is gripping through the gripping action. That is to say, in the case of this subject 5, his or her left fingers are the affected region, and the rehabilitation support system 100 is used to support the subject 5 in his or her rehabilitation to recover the ability to do voluntary movement, namely, the ability to grip something with his or her left fingers and the ability to stretch his or her left fingers. Actually, however, the rehabilitation support system 100 does not directly support the subject 5 in his or her gripping action but indirectly supports him or her in the attempt (rehabilitation) to recover the ability to grip something by assisting the subject 5 with his or her action of stretching fingers.

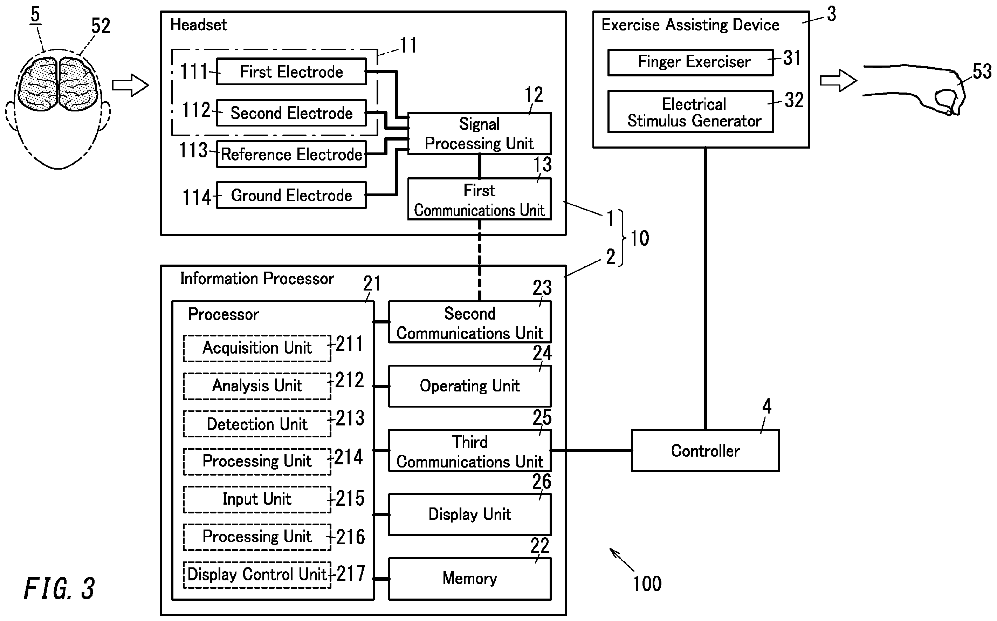

Thus, the rehabilitation support system 100 supports the subject 5, who is doing the stretching action as the voluntary movement, in his or her voluntary movement by making the exercise assisting device 3 worn by the subject 5 on his or her left hand apply at least one of a mechanical stimulus or an electrical stimulus to the subject's 5 left fingers 53. Specifically, the exercise assisting device 3 includes a finger exerciser 31 and an electrical stimulus generator 32 as shown in FIG. 3.

The finger exerciser 31 is a device for moving four fingers 53 (namely, the second to fifth fingers), except the first finger (thumb), by holding the four fingers 53 and applying a mechanical stimulus (external force) to these four fingers 53. The finger exerciser 31 includes a power source such a motor or a solenoid and is configured to move the four fingers 53 by transmitting the force generated by the power source to the four fingers 53. The finger exerciser 31 is able to do two types of operations, namely, an "opening operation" of moving the four fingers 53 held away from the first finger (i.e., stretching the four fingers 53) and a "closing operation" of moving the four fingers 53 toward the first finger (i.e., making the fingers 53 grip something). The finger exerciser's 31 opening operation assists the subject 5 with his or her stretching action, and the finger exerciser's 31 closing operation assists the subject 5 with his or her gripping action.

The electrical stimulus generator 32 is a device for applying an electrical stimulus to the subject's 5 region for moving his or her fingers 53. In this case, the subject's 5 region for moving his or her fingers 53 includes a region corresponding to at least one of a muscle or a nerve of the subject's 5 fingers 53. For example, the subject's 5 region for moving his or her fingers 53 may be a part of the subject's 5 right or left arm. The electrical stimulus generator 32 includes a pad to be attached to the subject's 5 body (such as his or her right or left arm). The electrical stimulus generator 32 applies a stimulus to the region for moving the fingers 53 by applying an electrical stimulus (in the form of an electrical current) from the pad to the subject's 5 body.

The controller 4 controls the exercise assisting device 3 in accordance with the electroencephalogram information acquired by the electroencephalogram measurement system 10. In this embodiment, the controller 4 is electrically connected to the information processor 2 of the electroencephalogram measurement system 10 and the exercise assisting device 3. A power cable for supplying operating power to the exercise assisting device 3 and the controller 4 is connected to the controller 4. The controller 4 includes a driver circuit for driving the finger exerciser 31 of the exercise assisting device 3 and an oscillator circuit for driving the electrical stimulus generator 32. The controller 4 receives a control signal from the information processor 2 via wired communication, for example.

On receiving a first control signal from the information processor 2, the controller 4 makes its driver circuit drive the finger exerciser 31 of the exercise assisting device 3, thereby controlling the exercise assisting device 3 such that the finger exerciser 31 performs the "opening operation." Also, on receiving a second control signal from the information processor 2, the controller 4 makes its driver circuit drive the finger exerciser 31 of the exercise assisting device 3, thereby controlling the exercise assisting device 3 such that the finger exerciser 31 performs the "closing operation." Furthermore, on receiving a third control signal from the information processor 2, the controller 4 makes its oscillator circuit drive the electrical stimulus generator 32 of the exercise assisting device 3, thereby controlling the exercise assisting device 3 such that an electrical stimulus is applied to the subject's 5 body.

This allows the controller 4 to control the exercise assisting device 3 based on the electroencephalogram information acquired by the electroencephalogram measurement system 10 by controlling the exercise assisting device 3 in accordance with the control signals supplied from the electroencephalogram measurement system 10. Optionally, the controller 4 may also control the exercise assisting device 3 such that the finger exerciser 31 performs the "opening operation" and the "closing operation" in response to a turn of an operating switch provided for the controller 4.

### (3) Electroencephalogram measurement system

The electroencephalogram measurement system 10 according to this embodiment will be described in further detail.

As shown in FIG. 3, the electroencephalogram measurement system 10 includes: the headset 1 to be worn by the subject 5 on his or her head 52; and the information processor 2 including, as a major constituent element, a computer system such as a personal computer.

The headset 1 includes: the electrode unit 11; a signal processing unit 12; and a first communications unit 13. The headset 1 may be driven by a battery, for example, which supplies power for operating the signal processing unit 12 and the first communications unit 13.

The electrode unit 11 includes electrodes for measuring and recording the subject's 5 electroencephalogram (in the form of an electroencephalogram signal), which may be silver-silver chloride electrodes, for example. The electrode unit 11 may also be made of gold, silver, or platinum, for example. The electrode unit 11 includes a first electrode 111 and a second electrode 112. In this embodiment, the region of interest 51 set on the surface of the subject's 5 head 52 includes a first region of interest 511 and a second region of interest 512 as shown in FIG. 2. The first electrode 111 corresponds to, and is placed on, the first region of interest 511. The second electrode 112 corresponds to, and is placed on, the second region of interest 512. Specifically, the first region of interest 511 and the second region of interest 512 are arranged in this order on a line segment connecting the median center of the head 52 to the subject's 5 right ear such that the first region of interest 511 is located closer to the median center than (i.e., located over) the second region of interest 512 is.

Also, in this embodiment, the headset 1 further includes a reference electrode 113 and a ground electrode 114. The reference electrode 113 is an electrode for measuring the reference potential of the electroencephalogram signal to be measured by each of the first electrode 111 and the second electrode 112. The reference electrode 113 is arranged behind either the right ear or the left ear of the head 52. Specifically, the reference electrode 113 is arranged behind the ear, on which the first electrode 111 and the second electrode 112 are placed, on the head 52. In this embodiment, the first electrode 111 and the second electrode 112 are placed on the right surface of the head 52, and therefore, the reference electrode 113 is arranged behind the right ear. On the other hand, the ground electrode 114 is arranged behind the right or left ear, on which the reference electrode 113 is not placed, on the head 52. In this embodiment, the reference electrode 113 is arranged behind on the right ear, and therefore, the ground electrode 114 is arranged behind the left ear. Each of the reference electrode 113 and the ground electrode 114 is electrically connected to the body 15 of the headset 1 via electric wires 16 (see FIG. 2) and is attached to the surface of the head 52 (i.e., the scalp). Optionally, the reference electrode 113 and the ground electrode 114 do not have to be placed behind the right or left ear but may also be placed on the right or left earlobe. In any case, the region behind the right or left ear and the right or left earlobe are regions of the head that are not easily affected by a bioelectric potential deriving from the brain activity. In other words, the reference electrode 113 and the ground electrode 114 are suitably arranged in such regions of the head that are not easily affected by a bioelectric potential deriving from the brain activity.

The signal processing unit 12 includes a processor and its peripheral circuits. The signal processing unit 12 is electrically connected to the electrode unit 11, the reference electrode 113, and the ground electrode 114 to perform signal processing on the electroencephalogram signal (electrical signal) supplied from the electrode unit 11 and generate electroencephalogram information. That is to say, the headset 1 detects the subject's 5 electroencephalogram by having the electrode unit 11 measure the activity potential of the subject's 5 brain, to make the signal processing unit 12 generate electroencephalogram information representing the electroencephalogram. The signal processing unit 12 includes at least an amplifier for amplifying the electroencephalogram signal and an A/D converter for converting an analog electroencephalogram signal into a digital signal, and outputs, as the electroencephalogram information, the amplified digital electroencephalogram signal.

In addition, the signal processing unit 12 further performs the function as an impedance measurement unit 121. The impedance measurement unit 121 measures the impedance value between the electrode unit 11 and the subject's 5 body (such as his or her head 52 where the electrode unit 11 is placed). Then, the signal processing unit 12 transmits impedance information, representing the impedance value measured by the impedance measurement unit 121, to the information processor 2 via the first communications unit 13.

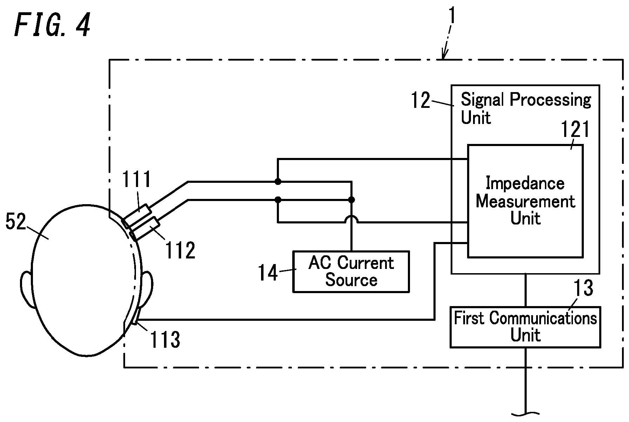

In the electroencephalogram measurement system 10 according to this embodiment, the headset 1 includes an AC current source 14 for measuring the impedance value between the electrode unit 11 and the subject's 5 body as shown in FIG. 4. The signal processing unit 12, the first communications unit 13, the AC current source 14, and other circuit components are housed in the body 15 of the headset 1.

The AC current source 14 allows an alternating current with a predetermined frequency and a predetermined current value to flow between the first electrode 111 and the reference electrode 113 and between the second electrode 112 and the reference electrode 113. The impedance measurement unit 121 includes an A/D converter, for example. The impedance measurement unit 121 detects the value of a voltage generated between the first electrode 111 and the reference electrode 113 and the value of a voltage generated between the second electrode 112 and the reference electrode 113. The impedance measurement unit 121 measures, based on the value of the voltage between the first electrode 111 and the reference electrode 113, the impedance value between the first electrode 111 and the body, including the impedance value between a region of contact where the first electrode 111 is in contact with the body and the reference electrode 113. In addition, the impedance measurement unit 121 also measures, based on the value of the voltage between the second electrode 112 and the reference electrode 113, the impedance value between the second electrode 112 and the body, including the impedance value between a region of contact where the second electrode 112 is in contact with the body and the reference electrode 113. Note that the impedance measurement unit 121 does not have to measure the impedance in this manner but may adopt any other method for measurement.

In this case, the frequency band of the alternating current supplied by the AC current source 14 is different from the frequency band where the event-related desynchronization causes a decline in power (e.g., a frequency band from 8 Hz to less than 30 Hz). The AC current source 14 is configured to supply an alternating current falling within a frequency band from a few hundred Hz to several kHz. Dividing the frequency band in this manner allows a filter circuit to separate an electrical signal representing an electroencephalogram to be detected by the electrode unit 11 from an alternating current flowing to measure the impedance value. This reduces the chances of the alternating current, flowing to measure the impedance value, affecting the electrical signal representing the electroencephalogram to be detected by the electrode unit 11, thus enabling the electrode unit 11 to measure the electroencephalogram information accurately.

The first communications unit 13 has the capability of communicating with the information processor 2. The first communications unit 13 transmits, to the information processor 2, at least the electroencephalogram information generated by the signal processing unit 12 and the impedance information representing the impedance value measured by the impedance measurement unit 121. In this embodiment, the first communications unit 13 is able to communicate with the information processor 2 bidirectionally. The communications protocol of the first communications unit 13 may be wireless communication compliant with the Bluetooth^{®} standard. In this electroencephalogram measurement system 10, the electroencephalogram information and the impedance information are transmitted, as needed, from the first communications unit 13 to the information processor 2 in the acquisition mode, for example.

The information processor 2 includes, as its major constituent element, a computer system including a processor 21 and a memory 22. The information processor 2 further includes a second communications unit 23, an operating unit 24, a third communications unit 25, and a display unit 26.

The second communications unit 23 has the capability of communicating with (the first communications unit 13 of) the headset 1. The second communications unit 23 receives at least the electroencephalogram information and the impedance information from the headset 1. In this embodiment, the second communications unit 23 is able to communicate with the headset 1 bidirectionally. The second communications unit 23 receives, as needed, the electroencephalogram information and impedance information, sampled at a sample frequency of about 200 Hz, for example, from the headset 1.

The third communications unit 25 has the capability of communicating with the controller 4. The third communications unit 25 transmits at least a control signal to the controller 4. The communications protocol of the third communications unit 25 is a wired communication compliant with the universal serial bus (USB) standard.

In this embodiment, the information processor 2 is equipped with a touchscreen panel display, which functions as the operating unit 24 and the display unit 26. Thus, the information processor 2 determines, when the operating unit 24 detects any of various types of operations (including tapping, swiping, and dragging) on buttons and other objects on the screen of the display unit 26, that the buttons and other objects should have been operated. That is to say, the operating unit 24 and the display unit 26 function as a user interface that not only displays various types of information thereon but also accepts operating commands entered by either the subject 5 or a medical staff. Note that the operating unit 24 does not have to be implemented as a touchscreen panel display but may also be implemented as a keyboard, a pointing device, a mechanical switch, or any other type of input device.

The processor 21 has various functions serving as an acquisition unit 211, an output unit 212, an electroencephalogram information presentation unit 213, a decision unit 214, a decision presentation unit 215, a processing unit 216, and a display control unit 217. The respective functions of the acquisition unit 211, the output unit 212, the electroencephalogram information presentation unit 213, the decision unit 214, the decision presentation unit 215, the processing unit 216, and the display control unit 217 are performed by having a program stored in the memory 22 executed by the processor 21.

The acquisition unit 211 acquires the impedance information, representing the impedance value measured by the impedance measurement unit 121, from the headset 1 via the second communications unit 23. The impedance measurement unit 121 measures the impedance value between the electrode unit 11 placed on the region of interest 51 forming part of the subject's 5 head 52 and the subject's 5 body.

The acquisition unit 211 also acquires electroencephalogram information representing an electroencephalogram obtained by the electrode unit 11 placed on the region of interest 51 that forms part of the subject's 5 head 52. That is to say, the acquisition unit 211 acquires, from the headset 1 and via the second communications unit 23, the electroencephalogram information representing the electroencephalogram obtained by the electrode unit 11 of the headset 1. Specifically, in this embodiment, the acquisition unit 211 acquires first electroencephalogram information representing the electroencephalogram obtained by the first electrode 111 and second electroencephalogram information representing the electroencephalogram obtained by the second electrode 112. That is to say, in this embodiment, the electrode unit 11 includes the first electrode 111 and the second electrode 112. Thus, the acquisition unit 211 regards the electroencephalogram information collected by the first electrode 111 and the electroencephalogram information collected by the second electrode 112 as the first electroencephalogram information and the second electroencephalogram information, respectively. In this embodiment, the acquisition unit 211 acquires electroencephalogram information in a digital form and stores, in the memory 22, the electroencephalogram information thus acquired. At this time, the time-series data of the electroencephalogram information collected by the electroencephalogram measurement system 10 since the beginning through the end of the training period is stored in the memory 22.

The output unit 212 outputs the impedance value measured acquired by the acquisition unit 211. The measuring result of the impedance value may be output from the output unit 212 as a text message displayed, a verbal message emitted, a page printed out, a file written into a non-transitory storage medium, or data transmitted to a telecommunications device, for example. In this embodiment, the output unit 212 outputs the impedance value measured to the display control unit 217 such that the impedance value measured is displayed on the display unit 26. In this embodiment, the output unit 212 displays, as a numerical value, the impedance value measured on the display unit 26. Optionally, the number of significant digits of the numerical value displayed on the display unit 26 may be changed. For example, when either the medical staff or the subject 5 enters setting information to set the number of significant digits using the operating unit 24, the setting information is output from the operating unit 24 to the output unit 212. The output unit 212 outputs, based on the setting information entered through the operating unit 24, the impedance value measured to the display control unit 217 such that the impedance value measured is displayed as a numerical value with the number of significant digits specified by the setting information.

The electroencephalogram information presentation unit 213 presents the electroencephalogram information acquired by the acquisition unit 211. The electroencephalogram information presentation unit 213 may present the electroencephalogram information as a text message displayed, a page printed out, a file written into a non-transitory storage medium, or data transmitted to a telecommunications device, for example. In this embodiment, the electroencephalogram information presentation unit 213 outputs the electroencephalogram information to the display control unit 217 such that the electroencephalogram information is displayed on the display unit 26.

The decision unit 214 determines, based on the impedance value measured as acquired by the acquisition unit 211, whether the electroencephalogram is sensed by the electrode unit 11 in good condition or not. For example, the decision unit 214 may determine, by comparing the impedance value measured as acquired by the acquisition unit 211 with a predetermined threshold value (of 50 kΩ, for example), whether the electroencephalogram is sensed by the electrode unit 11 in good condition or not. When finding the impedance value measured as acquired by the acquisition unit 211 less than the predetermined threshold value, the decision unit 214 determines the condition of contact between the electrode unit 11 and the region of interest, i.e., the condition in which the electroencephalogram is sensed by the electrode unit 11, to be good. On the other hand, when finding the impedance value measured as acquired by the acquisition unit 211 equal to or greater than the predetermined threshold value, the decision unit 214 determines the condition of contact between the electrode unit 11 and the region of interest, i.e., the condition in which the electroencephalogram is sensed by the electrode unit 11, to be bad.

The decision presentation unit 215 presents the decision made by the decision unit 214. The decision may be presented by the decision presentation unit 215 as, for example, a text message displayed, a verbal message emitted, a page printed out, a file written into a non-transitory storage medium, or data transmitted to a telecommunications device. In this embodiment, the decision presentation unit 215 outputs the decision to the display control unit 217 such that the decision made by the decision unit 214 is displayed on the display unit 26.

The processing unit 216 subjects the electroencephalogram information acquired by the acquisition unit 211 to various types of processing or displays the electroencephalogram information. In this embodiment, the processing unit 216 performs the processing of detecting an electroencephalogram with a characteristic variation that may arise when the subject 5 plans to do (or imagines doing) some voluntary movement and the processing of generating a control signal for controlling the exercise assisting device 3 based on the result of detection of the electroencephalogram.

The display control unit 217 makes the display unit 26 display the impedance value measured as provided by the output unit 212. The display control unit 217 has the capability of controlling the display unit 26 and may make the display unit 26 display various types of content thereon. The display control unit 217 makes the display unit 26 display at least the impedance value measured as provided by the output unit 212, the electroencephalogram information presented by the electroencephalogram information presentation unit 213, and the decision presented by the decision presentation unit 215. Note that the display control unit 217 makes the display unit 26 display, based on the impedance value measured as provided by the output unit 212, the impedance value measured as a numerical value with the number of significant digits specified by the setting information.

### (4) Operation

Next, it will be described how to use this rehabilitation support system 100 including the electroencephalogram measurement system 10 according to this embodiment.

In the following description of this embodiment, it will be described how the rehabilitation support system 100 supports the subject 5 in his or her voluntary movement (i.e., the stretching action) to be done by the subject 5 in order to release a peg 101 (see FIG. 1) from his or her left hand by stretching the fingers 53 from a position where he or she is gripping the peg 101 with his or her left fingers. In this embodiment, a medical staff supports the subject 5 in the action of stretching his or her fingers using the rehabilitation support system 100.

First of all, as a preparation process, the display control unit 217 of the information processor 2 makes the display unit 26 display an input screen prompting the user to enter information about the subject 5 who is going to undergo rehabilitation (such as the name of the subject 5, the target region of the rehabilitation, and other pieces of information). For example, when a medical staff enters, using the operating unit 24, information about the subject 5 through the input screen displayed on the display unit 26, the display control unit 217 of the information processor 2 makes the display unit 26 display an instruction screen to instruct the subject 5 to wear the headset 1 and the exercise assisting device 3.

In accordance with the instructions displayed on the display unit 26, the medical staff helps the subject 5 wear the headset 1 on his or her head 52 and the exercise assisting device 3 on his or her left hand. In this case, the subject 5 is made to wear the headset 1 on his or her head 52 such that at least the electrode unit 11 is brought into contact with a part of the right surface of the subject's 5 head 52, which constitutes the region of interest 51. The exercise assisting device 3 is worn by the subject 5 so as to hold at least the four fingers 53 (i.e., the second to fifth fingers), except the first finger (thumb), of the subject's 5 left hand and attach the pad to the subject's 5 left arm. The headset 1 and the exercise assisting device 3 may be firmly fixed as appropriate so as not to be displaced or come loose during the rehabilitation. In the preparation process, the subject's 5 four fingers 53 are held by the finger exerciser 31 of the exercise assisting device 3 to make the subject 5 keep gripping the peg 101 with his or her left fingers. The subject 5 may be equipped with the headset 1 and the exercise assisting device 3 by either the subject 5 him- or herself or a medical staff.

When the preparation is done to make the headset 1 and the information processor 2 ready to communicate with each other, the impedance value measured by the impedance measurement unit 121 of the headset 1 may be acquired by the information processor 2. That is to say, the electroencephalogram measurement system 10 may acquire, at the information processor 2, the impedance value measured between the electrode unit 11 placed on the region of interest 51 that forms part of the subject's 5 head 52 and the subject's 5 body.

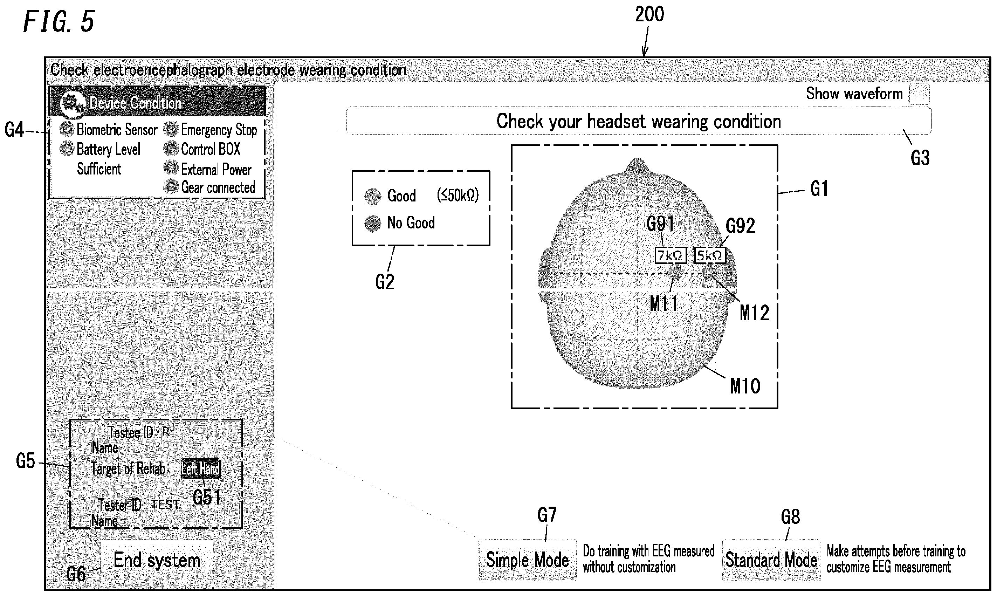

When the acquisition unit 211 of the information processor 2 acquires the impedance value measured, the decision unit 214 compares the impedance value measured with a predetermined threshold value. The output unit 212 of the information processor 2 makes the display unit 26 display a check screen 200 (see FIG. 5) showing the impedance value measured. In addition, the decision presentation unit 215 controls the display unit 26 to display the decision made by the decision unit 214 on the check screen 200 (see FIG. 5). Note that in the example illustrated in FIG. 5, the one-dot chain indicating the region and the reference signs are just shown there for the sake of convenience and are actually not displayed on the display unit 26.

The check screen 200 includes an impedance value display area G1, a legend display area G2, an operation guidance area G3, a device condition display area G4, a status display area G5, an end button G6, a simple mode select button G7, and a standard mode select button G8.

In the impedance value display area G1, displayed is a figure M10 representing a head. The figure M10 is an image generated by using a computer in this example but may also be a picture or a photo. In addition, in the impedance value display area G1, marks M11 and M12 representing the first electrode 111 and the second electrode 112, respectively, are also displayed at respective locations corresponding to the first electrode 111 and the second electrode 112 in the figure M10 representing the head. That is to say, the decision presentation unit 215 indicates the respective locations of the electrode unit 11 (including the first electrode 111 and the second electrode 112) on the figure M10 representing the head. Furthermore, in the impedance value display area G1, further provided are display areas G91 and G92 over the marks M11 and M12 representing the first electrode 111 and the second electrode 112, respectively. In the example shown in FIG. 5, the impedance value measured (e.g., 7 kΩ) between the first electrode 111 and the body is shown in the display area G91, and the impedance value measured (e.g., 5 kΩ) between the second electrode 112 and the body is shown in the display area G92.

In FIG. 5, each impedance value is indicated as a numerical value, of which the number of significant digits is one. However, the number of significant digits of the impedance value that the output unit 212 makes the display unit 26 display is changeable. For example, when a medical staff or any other person enters setting information specifying the number of significant digits of the numerical value through the operating unit 24, the output unit 212 may change, according to the setting information entered through the operating unit 24, the number of significant digits of the impedance value displayed in the display unit 26. More specifically, if the medical staff or the subject 5 enters, through the operating unit 24, setting information to specify the number of significant digits as three digits (e.g., two digits below the decimal point), then the output unit 212 outputs the impedance value measured as a numerical value, of which the number of significant digits is three in accordance with the setting information, to the display control unit 217. In response, the display control unit 217 displays the impedance value measured as a numerical value with three significant digits on the display unit 26. For example, in that case, the impedance value measured between the first electrode 111 and the body may be indicated as 7.31 kΩ in the display area G91, and the impedance value measured between the second electrode 112 and the body may be indicated as 5.26 kΩ in the display area G92. This allows the medical staff or the subject 5 to adjust the condition of contact between the electrode unit 11 and the body while checking the impedance value measured and shown on the display unit 26.

In this case, if the absolute value of the difference between the impedance value between the first electrode 111 and the body and the impedance value between the second electrode 112 and the body increases, then the quality of an electroencephalogram signal measured by using the first electrode 111 and the second electrode 112 may deteriorate. That is why the condition of contact between the first electrode 111 and the second electrode 112 and the body is suitably adjusted so as to decrease the absolute value of the difference of the impedance value between the first electrode 111 and the body from the impedance value between the second electrode 112 and the body. As described above, if the impedance value is displayed as a numerical value with three significant digits (of which two digits are below the decimal point), then either the medical staff or the subject may adjust the condition of contact between the plurality of electrode units 11 and the body with a precision of three significant digits (of which two digits are below the decimal point). This improves the quality of the electroencephalogram signal to be measured by using the first electrode 111 and the second electrode 112. Note that the number of significant digits of the impedance value between the electrode unit 11 and the body does not have to be one or three, but may also be two, four, or more

Furthermore, the setting information for setting the number of significant digits does not have to be information entered through the operating unit 24 but may also be determined by the processor of the information processor 2 according to the skill level of the medical staff that uses the electroencephalogram measurement system 10 or any other factor. For example, the processor 21 of the information processor 2 may generate the setting information such that the higher the skill level of the medical staff is, the larger the number of significant digits of the impedance value displayed on the display unit 26 is. Increasing the number of significant digits of the numerical value displayed on the display unit 26 as the skill level of the medical staff rises in this manner allows the medical staff to more finely adjust the condition of contact between the electrode unit 11 and the subject's 5 body. Furthermore, in the example illustrated in FIG. 5, the impedance value between the first electrode 111 and the body and the impedance value between the second electrode 112 and the body are displayed in kΩ. Alternatively, these impedance values may also be displayed in Ω. For example, the output unit 212 may display the impedance values in the unit specified by either the medical staff or the subject 5 through the operating unit 24 or may also change the unit of the impedance values displayed appropriately according to the impedance values measured and the number of significant digits thereof.

Also, the decision presentation unit 215 controls the display control unit 217 so as to change the color of the marks M11, M12 displayed according to the decision made about whether the electroencephalogram is sensed by the electrode unit 11 in good condition or not. That is to say, the decision presentation unit 215 indicates, by the color, whether the electroencephalogram is sensed in good condition or not, according to the decision made by the decision unit 214. Likewise, the decision presentation unit 215 may also change a mode of display (such as the display color) of the impedance value measured depending on whether the electroencephalogram is sensed in good condition or not, according to the decision made by the decision unit 214.

The legend display area G2 is an area for providing description about the display mode of the marks M11 and M12. In this embodiment, the decision presentation unit 215 changes the mode of display (such as the display color) of the marks M11 and M12 depending on whether the electroencephalogram is determined to be sensed in good condition or not. Thus, in the legend display area G2, the two display modes of the marks M11 and M12 are presented along with their description. The decision presentation unit 215 controls the display control unit 217 so as to display the marks M11 and M12 in blue when the electroencephalogram is determined to be sensed in good condition and display the marks M11 and M12 in red when the electroencephalogram is determined to be sensed in bad condition.

The operation guidance area G3 is an area for displaying text information as a guidance for operating the rehabilitation support system 100 properly. In the example illustrated in FIG. 5, text information that says "check your headset wearing condition" is displayed in the operation guidance area G3. The information displayed on the operation guidance area G3 varies according to the operating state of the rehabilitation support system 100.

The device condition display area G4 is an area for displaying the respective conditions of various devices that form the rehabilitation support system 100. In the device condition display area G4, the condition of connection between the information processor 2 and the headset 1, the battery level of the headset 1, and other conditions are indicated by icons such as open circles (O) and crosses (X).

The status display area G5 is an area for displaying the identification information (testee ID) and name of the subject 5, and the identification information (tester ID) and name of the medical staff attending him or her. In addition, in the status display area G5, a target icon G51, indicating whether the "target of rehabilitation" is "left hand" or "right hand" is further displayed. In the example illustrated in FIG. 5, the target icon G51 indicates, as text information, that the target of rehabilitation is the "left hand."

The end button G6 is a button to be tapped to end the training. Tapping the end button G6 brings not only the training by the rehabilitation support system 100 but also the electroencephalogram measurement by the electroencephalogram measurement system 10 to an end.

The simple mode select button G7 is a button to be tapped to start measuring electroencephalogram information with the electroencephalogram measuring condition and other conditions unchanged from standard settings. Tapping the simple mode select button G7 causes training by the rehabilitation support system 100 to be started with the electroencephalogram measuring condition and other conditions unchanged from standard settings.

The standard mode select button G8 is a button to be tapped to start measuring electroencephalogram information with the electroencephalogram measuring condition and other conditions customized. Tapping the standard mode select button G8 causes a screen for customizing the electroencephalogram measuring condition and other conditions to be displayed. Entering customized electroencephalogram measuring condition and other conditions allows the training by the rehabilitation support system 100 to be started.

In the impedance value display area G1 of the check screen 200, the impedance values measured, and decision made by the decision unit 214 are displayed for the electrode unit 11 including the first electrode 111 and the second electrode 112. Therefore, if the decision made by the decision unit 214 is "bad" for at least one of the first electrode 111 or the second electrode 112, either the medical staff or the subject 5 may rectify the wearing condition of the electrode that has turned out to be worn improperly out of the first electrode 111 and the second electrode 112. This allows the rehabilitation support system 100 to start training for the subject 5 with the impedance value between the first electrode 111 and the second electrode 112 and the body kept less than a predetermined threshold value.

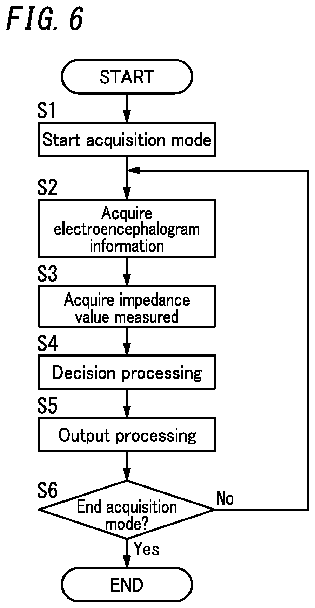

In this case, on the check screen 200 shown in FIG. 5, tapping the simple mode select button G7 causes the processor 21 of the information processor 2 to receive a signal from the operating unit 24 to start a training process for performing training by measuring the electroencephalogram. That is to say, the rehabilitation support system 100 starts the training process. Next, it will be described with reference to the flowchart of FIG. 6 how the electroencephalogram measurement system 10 performs an acquisition mode of operation for acquiring electroencephalogram information during the training process by the rehabilitation support system 100.

When the processor 21 of the information processor 2 starts performing the acquisition mode of operation (in Step S1), the acquisition unit 211 of the processor 21 acquires electroencephalogram information from the headset 1 via the second communications unit 23 (in Step S2). In addition, the acquisition unit 211 also acquires, from the headset 1 via the second communications unit 23, the impedance values of the first electrode 111 and the second electrode 112 that have been measured by the impedance measurement unit 121 (in Step S3). In this case, the processing step of acquiring the electroencephalogram information and the processing step of acquiring the impedance values measured may be performed in any order without limitation. That is to say, the electroencephalogram information may be acquired after the acquisition unit 211 has acquired the impedance values measured. Note that the signal processing unit 12 does not have to perform the processing of measuring the electroencephalogram and the processing of measuring the impedance values at the same timing. That is to say, the timing for the electrode unit 11 to measure and record the electroencephalogram and the timing for the electrode unit 11 to measure the impedance values may be somewhat different from each other, as long as these two timings may be regarded as substantially the same. In other words, in this electroencephalogram measurement system 10, measurement and recording of the electroencephalogram by the electrode unit 11 and measurement of the impedance values by the impedance measurement unit 121 just need to be performed in parallel with each other.

When the acquisition unit 211 acquires the electroencephalogram information, the electroencephalogram information presentation unit 213 outputs the electroencephalogram information to the display control unit 217. In response, the display control unit 217 displays the electroencephalogram information on the display unit 26. In addition, when the acquisition unit 211 acquires the electroencephalogram information, the processor 21 stores (accumulates), in the memory 22, the electroencephalogram information acquired by the acquisition unit 211.

Furthermore, when the acquisition unit 211 acquires the impedance values measured with the first electrode 111 and the second electrode 112, the decision unit 214 compares the impedance values measured with the first electrode 111 and the second electrode 112 with a predetermined threshold value. Then, based on the result of comparison between the impedance values measured with the first electrode 111 and the second electrode 112 and the predetermined threshold value, the decision unit 214 determines whether the electroencephalogram is sensed by the first electrode 111 and the second electrode 112 in good condition or not (in Step S4).

Next, the output unit 212 outputs the impedance values measured to the display control unit 217, which in turn has the impedance values measured displayed on the display unit 26 (in Step S5). Meanwhile, the decision presentation unit 215 outputs the decision made by the decision unit 214 to the display control unit 217, which in turn has the decision made by the decision unit 214 displayed on the display unit 26.

Thereafter, the processor 21 determines whether or not the acquisition mode of operation has ended (in Step S6). If the acquisition mode of operation has not ended yet (if the answer is NO in Step S6), the process goes back to the processing step S2 to have the same series of processing steps S2-S5 performed all over again. On the other hand, if the acquisition mode of operation has ended in Step S6 (if the answer is YES in Step S6), then the processor 21 ends the processing of measuring electroencephalogram information and the impedance values.

In this case, the rehabilitation support system 100 according to this embodiment supports the subject 5 in his or her rehabilitation in accordance with the electroencephalogram information that the electroencephalogram measurement system 10 has acquired through the acquisition mode of operation during the training process.

The processing unit 216 of the processor 21 generates a power spectrum of the electroencephalogram by carrying out a time frequency analysis on the electroencephalogram information acquired by the acquisition unit 211 in the acquisition mode. This allows the electroencephalogram measurement system 10 to detect an electroencephalogram with a characteristic variation that may arise when the subject 5 is going to do the voluntary movement (i.e., when the subject 5 plans to do voluntary movement) by making the information processor 2 constantly monitor the data of the power spectrum.

The training period is subdivided into two periods, namely, a rest period and an exercise period. In each of the rest period and the exercise period, the subject 5 undergoes his or her rehabilitation in accordance with the instructions given by the rehabilitation support system 100. In this embodiment, the training period may be 10 seconds, and if the training period is evenly divided into two, then the first half of 5 seconds is supposed to be the "rest period" and the second half of 5 seconds is supposed to be the "exercise period."

In the rest period, the subject 5 puts his or her body at rest (i.e., does not plan to do (or imagine doing) any voluntary movement) to keep relaxed. At this time, the electroencephalogram measurement system 10 does not detect any electroencephalogram with a characteristic variation that may arise due to the event-related desynchronization when the subject 5 plans to do the voluntary movement.

Meanwhile, in the exercise period, the subject 5 plans to do (or imagines doing) the action of stretching the fingers 53 as a type of voluntary movement. At this time, the electroencephalogram measurement system 10 may detect an electroencephalogram with a characteristic variation that may arise due to the event-related desynchronization when the subject 5 plans to do the voluntary movement. In this embodiment, the processing unit 216 of the electroencephalogram measurement system 10 detects such a characteristic variation in electroencephalogram by comparing an activation level with a threshold value and determining whether or not the activation level is greater than the threshold value. As used herein, the "activation level" refers to a value representing the magnitude of decline in power (i.e., power spectrum) in a particular frequency band. When the event-related desynchronization causes a decline in the power in the particular frequency band, the activation level exceeds the threshold value. Thus, the processing unit 216 of the electroencephalogram measurement system 10 detects the characteristic variation in electroencephalogram when finding the activation level greater than the threshold value.

In the processing unit 216 of this electroencephalogram measurement system 10, generation of a control signal for controlling the exercise assisting device 3 is triggered by the detection of the electroencephalogram with such a characteristic variation. This allows, when the subject 5 plans to do (or imagines doing) voluntary movement, the rehabilitation support system 100 to make the exercise assisting device 3 assist the subject 5 with the voluntary movement at the timing when a brain region, corresponding to the target region of the voluntary movement, is actually activated.

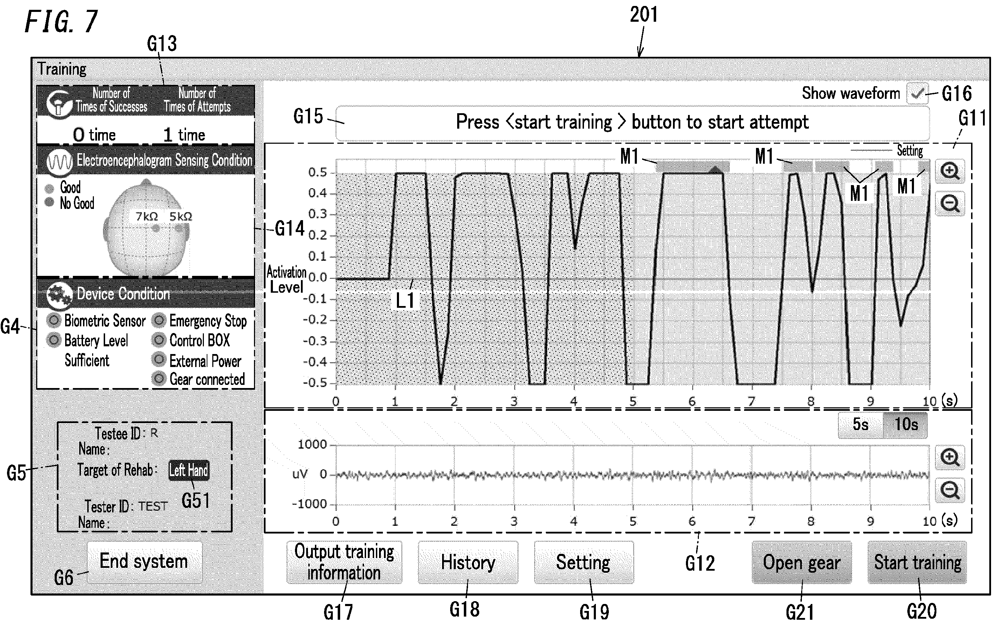

When the operation mode of the rehabilitation support system 100 is the training mode, a training screen 201 such as the one shown in FIG. 7 is displayed on the display unit 26. Note that in the example illustrated in FIG. 7, the one-dot chain indicating the region and the reference signs are just shown there for the sake of convenience and are actually not displayed on the display unit 26.

The training screen 201 includes an activation level display area G11, an electroencephalogram display area G12, a number of times display area G13, a sensing condition display area G14, an operation guidance area G15, a checkbox G16, a device condition display area G4, and a status display area G5. The training screen 201 further includes an end button G6, an output training information button G17, a history button G18, and a setting button G19.

The activation level display area G11 is an area for displaying an activation level graph. The activation level graph, of which the abscissa indicates the time (in seconds) and the ordinate indicates the activation level, displays a waveform showing how the activation level changes with time. In the activation level display area G11, the line L1 indicates a threshold value (as a setting). Also, in a period during which the activation level is greater than the threshold value, a decision mark M1 in the shape of a band is displayed over the activation level graph. In addition, the activation level graph suitably has two different background colors for the rest period (i.e., a period from 0 to 5 seconds) defining the first half of the training period and the exercise period (i.e., a period from 5 to 10 seconds) defining the second half of the training period, respectively.

The electroencephalogram display area G12 is an area for displaying the electroencephalogram measured by the headset 1. The electroencephalogram is displayed in the electroencephalogram display area G12 in real time in accordance with the electroencephalogram information transmitted from the headset 1 to the information processor 2. The electroencephalogram graph, of which the abscissa indicates the time (in seconds) and the ordinate indicates the potential, displays a waveform indicating how the electroencephalogram changes with time.

The start training button G20 is a button to be tapped to get the training started. Tapping the start training button G20 causes the training by the rehabilitation support system 100 to be started and also causes the electroencephalogram measurement system 10 to start measuring the electroencephalogram.

The gear operating button G21 is a button allowing the subject 5 to operate the exercise assisting device 3. Tapping the gear operating button G21 causes the electroencephalogram measurement system 10 to output a control signal to have the operation of closing or opening the exercise assisting device 3 performed. In this example, text information such as "close gear" or "open gear" is displayed on the gear operating button G21. Tapping the gear operating button G21 with "close gear" displayed causes the closing operation to be performed by the exercise assisting device 3. Tapping the gear operating button G21 with "open gear" displayed causes the opening operation to be performed by the exercise assisting device 3.

The number of times display area G13 is an area for displaying the number of times of successes and the number of times of attempts. As used herein, the "number of times of successes" indicates the number of times the electroencephalogram measurement system 10 has successfully detected the electroencephalogram, having the characteristic variation that may arise due to event-related desynchronization when the subject 5 plans to do the voluntary movement, during the exercise period of the training period. Also, as used herein, the "number of times of attempts" indicates the number of times the training by the rehabilitation support system 100 has been carried out.

The sensing condition display area G14 is an area for indicating a specific condition in which the electroencephalogram is sensed. In this embodiment, the electroencephalogram measurement system 10 measures the impedance value between the first electrode 111 and the subject's 5 body (i.e., his or her scalp) and the impedance value between the second electrode 112 and the subject's 5 body (i.e., his or her scalp), thereby determining, based on the impedance values measured, whether the electroencephalogram is sensed in good condition or not. In the example illustrated in FIG. 7, displayed in the sensing condition display area G14 are a text indicating the impedance values ("7 kΩ" and "5 kΩ" in FIG. 7) and markers indicating whether the electroencephalogram is sensed by the first electrode 111 and the second electrode 112 in good condition or not. Specifically, the markers on the illustration representing a human head change their colors depending on whether the electroencephalogram is sensed by each of the first electrode 111 and the second electrode 112 in good condition or not. In the example illustrated in FIG. 7, the impedance values are displayed as numerical values each having a single significant digit in the sensing condition display area G14. However, the number of significant digits of the impedance value may be changed in accordance with the setting information entered by either the medical staff or the subject 5 through the operating unit 24 as in the display on the check screen 200 shown in FIG. 5.

The capabilities of the device condition display area G4, the status display area G5, and the end button G6 are the same as their counterparts of the check screen 200 shown in FIG. 5, and description thereof will be omitted herein.

The operation guidance area G15 is an area for displaying text information providing guidance on how to operate the rehabilitation support system 100 properly. In the example illustrated in FIG. 7, the text information that says "press <start training > button to start attempt" is displayed in the operation guidance area G15. The message displayed in the operation guidance area G15 varies according to the operating state of the rehabilitation support system 100.

The checkbox G16 is an icon for switching the state of displaying the electroencephalogram in the electroencephalogram display area G12 into the state of displaying no electroencephalograms there, and vice versa. Tapping the checkbox G16 causes the display state to be switched alternately from the state where the electroencephalogram is displayed in the electroencephalogram display area G12 to the state where the electroencephalogram is not displayed in the electroencephalogram display area G12, and vice versa. While the checkbox G16 is checked off, the electroencephalogram is displayed in the electroencephalogram display area G12.

The output training information button G17 is a button for outputting training information including the result of rehabilitation. Tapping the output training information button G17 causes the training information to be output in any desired form. For example, the training information may be output as a text message displayed, a verbal message emitted, a page printed out, a file written into a non-transitory storage medium, or data transmitted to a telecommunications device, for example.

The history button G18 is a button allowing the user to refer to the history of the training information including the results of rehabilitation. Tapping the history button G18 changes the screens displayed on the display unit 26 of the information processor 2 from the training screen 201 to a history reference screen. On the history reference screen, at least the results of rehabilitations that the subject 5 has undergone so far are displayed.

The setting button G19 is a button for changing the operation mode of the rehabilitation support system 100 into the setting mode in which various types of settings are made about the rehabilitation support system 100. Tapping the setting button G19 changes the screens displayed on the display unit 26 of the information processor 2 from the training screen 201 to a setting screen where various types of settings may be made.

During the training process, the subject 5 undergoes the rehabilitation with the training screen 201 described above displayed on the display unit 26 of the information processor 2. That is to say, tapping the start training button G20 causes the training by the rehabilitation support system 100 to be started.

As soon as the training is started, the training time starts being counted, and the electroencephalogram measurement system 10 starts measuring the electroencephalogram specific to the subject 5. During the rest period (i.e., the period from 0 to 5 seconds) that is the first half of the training period, the subject 5 puts his or her body at rest in accordance with the guidance displayed in the operation guidance area G15 or the instruction given by the medical staff attending him or her. At this time, the activation level, the electroencephalogram, the impedance value measured, and the decision about the specific condition in which the electroencephalogram is sensed are displayed in real time on the training screen 201. During the rest period, however, the electroencephalogram measurement system 10 does not compare the activation level with the threshold value and does not detect an electroencephalogram with the characteristic variation caused by the event-related desynchronization, either.

On the other hand, during the exercise period (i.e., the period from 5 to 10 seconds) defining the second half of the training period, the subject 5 plans to do (or imagines doing) the action of stretching his or her fingers 53 as a voluntary movement in accordance with either the guidance displayed in the operation guidance area G15 or the instruction given by the medical staff attending him or her. At this time, the activation level, the electroencephalogram, the impedance value measured, and the decision about the specific condition in which the electroencephalogram is sensed are displayed in real time on the training screen 201. In addition, during the exercise period, the electroencephalogram measurement system 10 compares the activation level with the threshold value, thereby detecting an electroencephalogram with the characteristic variation caused by the event-related desynchronization. In this case, when the event-related desynchronization causes the activation level to exceed the threshold value (as indicated by the line L1 in FIG. 7), the band-shaped decision mark M1 appears over the activation level graph on the training screen 201.

More specifically, the processing unit 216 of the electroencephalogram measurement system 10 analyzes, as needed, the electroencephalogram information acquired by the acquisition unit 211, thereby obtaining power on a frequency band basis. In addition, the processing unit 216 of the electroencephalogram measurement system 10 calculates the activation level based on the power on the frequency band basis analyzed and compares the activation level with the threshold value. The processing unit 216 of the electroencephalogram measurement system 10 determines, when the event-related desynchronization causes a decline in the power in the particular frequency band to change the power in the particular frequency band from the rest range to the exercise range, that the activation level should have exceeded the threshold value.

In this case, the particular frequency band may be either a single frequency band (which may be either an α-wave range or a β-wave range) or a plurality of frequency bands (which may be both the α-wave range and the β-wave range). In a situation where the particular frequency band includes two frequency bands, for example, when the coordinate values defined by the powers of these two frequency bands make a transition from the rest range to the exercise range, the activation level exceeds the threshold value.

In addition, according to this embodiment, the processing unit 216 of the electroencephalogram measurement system 10 also has the capability of measuring the duration for which the activation level remains greater than the threshold value. In the training screen 201 illustrated as an example in FIG. 7, the length of each decision mark M1 corresponds to the length of the duration. When the activation level rises (i.e., changes) from a value equal to or less than the threshold value to a value greater than the threshold value, the processing unit 216 of the electroencephalogram measurement system 10 transmits the third control signal to the controller 4. Furthermore, when the duration reaches a prescribed amount of time (of 1 second, for example), the processing unit 216 of the electroencephalogram measurement system 10 transmits the first control signal to the controller 4.

As can be seen from the foregoing description, when the activation level exceeds the threshold value, the electrical stimulus generator 32 of the exercise assisting device 3 is driven and the exercise assisting device 3 applies an electrical stimulus to the subject's 5 body, thereby assisting the subject 5 with his or her voluntary movement (i.e., the stretching action in this case). Furthermore, when the activation level remains greater than the threshold value for the prescribed amount of time, the finger exerciser 31 of the exercise assisting device 3 is driven and the exercise assisting device 3 performs the "opening operation" with respect to the finger exerciser 31, thereby assisting the subject 5 with his or her voluntary movement (stretching action in this case).

As a result, in a situation where the subject 5 plans to do (or imagines doing) the voluntary movement, the exercise assisting device 3 assists the subject 5 with his or her voluntary movement (i.e., the action of stretching his or her left fingers 53) at the timing when a brain region corresponding to the target region of the voluntary movement is actually activated. At this time, the subject's 5 muscle and sensory nerve start their activity and the information is transmitted to the brain, thereby reconstructing the nerve system and achieving some rehabilitation effect. This allows the rehabilitation support system 100 to support the subject 5 in his or her rehabilitation by exercise therapy as well as in a situation where medical staff assists subjects 5 in various conditions and more effectively than in a situation where the subject 5 does the voluntary movement by him- or herself.

### (Variations)

The embodiment described above is only one of various embodiments of the present disclosure and may be readily modified or changed in various manners depending on a design choice or any other factor, without departing from the scope of the present disclosure. Also, the same function as that of the electroencephalogram measurement system 10 may also be implemented as a method for controlling the electroencephalogram measurement system, a (computer) program, or a non-transitory storage medium that stores the program thereon, for example. A method for controlling an electroencephalogram measurement system according to an aspect includes acquiring electroencephalogram information representing an electroencephalogram obtained by an electrode unit 11 that is placed on a region of interest 51 forming part of a subject's 5 head 52. The control method further includes measuring an impedance value between the electrode unit 11 and the subject's 5 body in an acquisition mode in which the electroencephalogram information is acquired; and outputting the impedance value measured. A (computer) program according to another aspect is designed to cause a computer system to carry out the method for controlling an electroencephalogram measurement system described above.

Next, variations of the embodiment described above will be enumerated one after another. Note that any of the variations to be described below may be combined as appropriate.

The electroencephalogram measurement system 10 according to the present disclosure includes a computer system. In that case, the computer system may include, as principal hardware components, a processor and a memory. The functions of the electroencephalogram measurement system 10 according to the present disclosure may be performed by making the processor execute a program stored in the memory of the computer system. The program may be stored in advance in the memory of the computer system. Alternatively, the program may also be downloaded through a telecommunications line or be distributed after having been recorded in some non-transitory storage medium such as a memory card, an optical disc, or a hard disk drive, any of which is readable for the computer system. The processor of the computer system may be made up of a single or a plurality of electronic circuits including a semiconductor integrated circuit (IC) or a largescale integrated circuit (LSI). As used herein, the "integrated circuit" such as an IC or an LSI is called by a different name depending on the degree of integration thereof. Examples of the integrated circuits include a system LSI, a very largescale integrated circuit (VLSI), and an ultra largescale integrated circuit (ULSI). Optionally, a field-programmable gate array (FPGA) to be programmed after an LSI has been fabricated or a reconfigurable logic device allowing the connections or circuit sections inside of an LSI to be reconfigured may also be adopted as the processor. Those electronic circuits may be either integrated together on a single chip or distributed on multiple chips, whichever is appropriate. Those multiple chips may be integrated together in a single device or distributed in multiple devices without limitation.

Also, in the embodiment described above, the plurality of constituent elements of the information processor 2 are integrated together in a single housing. However, this is not a configuration essential to the electroencephalogram measurement system 10. Alternatively, those constituent elements of the information processor 2 may be distributed in multiple different housings. Even when a plurality of constituent elements are distributed in multiple different housings, those constituent elements are still able to serve as the electroencephalogram measurement system 10 in conjunction with each other when connected together via a network such as the Internet. Still alternatively, at least some functions of the electroencephalogram measurement system 10 may be implemented as a server or a cloud computing system as well. Conversely, the plurality of functions distributed in multiple devices such as the headset 1 and the information processor 2 may be integrated as parts of the electroencephalogram measurement system 10, along with the other constituent elements of the electroencephalogram measurement system 10, in a single housing.

In the electroencephalogram measurement system 10 according to the present disclosure, measurement and recording of the electroencephalogram by the electrode unit 11 and measurement of the impedance values by the impedance measurement unit 121 are performed in parallel with each other. Optionally, the impedance measurement unit 121 may measure the impedance values intermittently in the acquisition mode. For example, the impedance measurement unit 121 may measure no impedance values while the electrode unit 11 is acquiring the electroencephalogram and may measure the impedance values while the electrode unit 11 is acquiring no electroencephalograms. Shifting the period during which the electrode unit 11 acquires the electroencephalogram from the period during which the impedance measurement unit 121 measures the impedance values in this manner improves the electroencephalogram measuring accuracy while keeping the electroencephalogram sensed in good condition by the electrode unit 11. In addition, the impedance measurement unit 121 does not have to constantly measure the impedance values. Alternatively, the impedance measurement unit 121 may measure the impedance values intermittently at predetermined time intervals during the training period.

In the electroencephalogram measurement system 10 according to the present disclosure, a plurality of electrode units 11 are provided and the impedance measurement unit 121 measures the impedance value between each of the plurality of electrode units 11 and the subject's 5 body. Optionally, the decision unit 214 may further determine, based on the relative relation in impedance value between the plurality of electrode units 11, whether the electroencephalogram is sensed by the plurality of electrode units 11 in good condition or not. As used herein, the relative relation in impedance value between the plurality of electrode units 11 may be, for example, the difference or ratio of the impedance value measured by one of the plurality of electrode units 11 to the impedance value measured by another one of the plurality of electrode units 11. For example, when finding the absolute value of the difference of the impedance value between the first electrode 111 and the body from the impedance value between the second electrode 112 and the body, for example, equal to or less than a predetermined tolerance, the decision unit 214 determines that the electroencephalogram should be sensed in good condition. Alternatively, when finding the ratio of the impedance value between the second electrode 112 and the body to the impedance value between the first electrode 111 and the body falling within a predetermined tolerance range, the decision unit 214 may also determine that the electroencephalogram should be sensed in good condition.

In this case, the decision made by the decision unit 214 is presented by the decision presentation unit 215 to the display control unit 217, which in turn has the decision presented by the decision presentation unit 215 displayed on the display unit 26. The decision made by the decision unit 214 includes the decision based on the magnitude of the impedance value measured with the first electrode 111 (hereinafter referred to as a "first decision") and the decision based on the relative relation of the impedance values between the plurality of electrode units 11 (hereinafter referred to as a "second decision"). The display control unit 217 has the first decision and the second decision displayed in mutually different modes on the display unit 26. For example, the decision presentation unit 215 may make, depending on whether the first decision is good or bad, the display modes of the marks M11 and M12 (such as the shapes, display colors and blinking states of the marks M11 and M12) representing the first electrode 111 and the second electrode 112, respectively, different from each other. In addition, the decision presentation unit 215 may make, depending on whether the second decision is good or bad, the display modes of the marks M11 and M12 (such as the shapes, display colors and blinking states of the marks M11 and M12) representing the first electrode 111 and the second electrode 112, respectively, different from each other. Furthermore, the decision presentation unit 215 may make the display modes of the marks M11 and M12 representing the first electrode 111 and second electrode 112, respectively, different from each other depending on whether the bad result is the first decision or the second decision. This allows either the medical staff or the subject 5 to learn, based on the display modes of the marks M11 and M12 displayed on the display unit 26, the first decision and the second decision. This allows the medical staff or the subject 5 to adjust the condition of contact between the plurality of electrode units 11 and the body so as to make not only the impedance values measured with the plurality of electrode units 11 but also the difference between the impedance values measured with the plurality of electrode units 11 as small as possible.

In the embodiment described above, the electroencephalogram measurement system 10 measures the impedance value between the first electrode 111 and the reference electrode 113 and the impedance value between the second electrode 112 and the reference electrode 113. Optionally, the electroencephalogram measurement system 10 may measure the impedance value between the first electrode 111 and the second electrode 112 as well.

Also, the electrode unit 11 does not have to be configured to come into contact with the surface of the subject's 5 head 52 (i.e., the scalp). Alternatively, the electrode unit 11 may also be configured to come into contact with the surface of the brain, for example.

Furthermore, the rehabilitation support system 100 does not always support the subject 5 in his or her rehabilitation to recover the proper function of his or her fingers but may also support the subject 5 in his or her rehabilitation for any other body region such as his or her shoulder(s), elbow(s), upper arm(s), waist, lower limbs, or upper limbs. The characteristic variation of an electroencephalogram that may arise when the subject 5 plans to do (or imagines doing) voluntary movement may vary depending on the target region of the rehabilitation or the type of the movement. For example, if an event-related synchronization (ERS) occurs when the subject 5 plans to do the voluntary movement, the power in a particular frequency band increases in the electroencephalogram representing a brain wave measured in the vicinity of the motor area during the voluntary movement. In that case, the electroencephalogram measurement system 10 detects, by seeing the power increase in the particular frequency band, the characteristic variation in electroencephalogram. Also, the target electroencephalogram to be detected by the electroencephalogram measurement system 10 does not have to be an electroencephalogram with a characteristic variation that may arise when the subject 5 plans to do voluntary movement. Alternatively, the target electroencephalogram to be detected may also be an electroencephalogram with a characteristic variation that may arise when the subject 5 is provided with a predetermined type of information. In this case, examples of the predetermined type of information include visual information (such as video and images) and auditory information (such as sounds or voices and music). That is to say, as long as the target electroencephalogram to be detected by the electroencephalogram measurement system 10 is an electroencephalogram with some characteristic variation, its characteristic variation may be caused by any factor.

Furthermore, the rehabilitation support system 100 does not have to be configured to apply either an electrical stimulus or a mechanical (or dynamic) stimulus to the subject 5 but may also be configured to apply a visual stimulus to the subject 5 by presenting virtual video to him or her. In that case, when the subject 5 plans to do the voluntary movement, the rehabilitation support system 100 presents virtual video, representing his or her affected region as if the affected region were functioning normally, to him or her at the timing when his or her brain region corresponding to the target region of the voluntary movement is actually activated. This also allows the rehabilitation support system 100 to support the subject 5 in his or her voluntary movement.

Furthermore, the exercise assisting device 3 and the controller 4 do not have to be provided separately from each other. Alternatively, the exercise assisting device 3 and the controller 4 may also be housed and integrated together in the same housing.

Furthermore, the method of communication between the headset 1 and the information processor 2 is supposed to be wireless communication in the embodiment described above but may also be wired communication or communications via a relay, for example. Furthermore, the method of communication between the controller 4 and the information processor 2 is supposed to be wired communication in the embodiment described above but may also be wireless communication or communication via a relay, for example.

Furthermore, the headset 1 does not have to be driven by a battery but the power to operate the signal processing unit 12, the first communications unit 13, and other components may also be supplied from the information processor 2, for example.

Furthermore, the information processor 2 does not have to be configured to acquire electroencephalogram information from the dedicated headset 1. Alternatively, the information processor 2 may also be configured to acquire electroencephalogram information from a general-purpose electroencephalograph, for example.

Furthermore, if one of two values (such as the impedance values measured for the first electrode 111 and the second electrode 112, respectively, and the threshold value) being compared with each other is "equal to or greater than" the other, the phrase "equal to or greater than" covers both a situation where these two values are equal to each other and a situation where one of the two values is greater than the other. However, this is only an example and should not be construed as limiting. The phrase "equal to or greater than" may also be a synonym of the phrase "greater than" that covers only a situation where one of the two values is greater than the other. That is to say, it is arbitrarily changeable, depending on selection of a threshold value or any preset value, whether or not the phrase "greater than" covers the situation where the two values are equal to each other. Therefore, from a technical point of view, there is no difference between the phrase "equal to or greater than" and the phrase "greater than." Similarly, the phrase "less than" may be a synonym of the phrase "equal to or less than."

### (Resume)

As can be seen from the foregoing description, an electroencephalogram measurement system (10) according to a first aspect includes an acquisition unit (211), an impedance measurement unit (121), and an output unit (212). The acquisition unit (211) acquires electroencephalogram information representing an electroencephalogram obtained by an electrode unit (11) that is placed on a region of interest (51) forming part of a subject's (5) head (52). The impedance measurement unit (121) measures an impedance value between the electrode unit (11) and the subject's (5) body in an acquisition mode in which the acquisition unit (211) acquires the electroencephalogram information. The output unit (212) outputs the impedance value measured.

This aspect allows the user to learn, based on the impedance value measured as provided by the output unit (212), a specific condition in which an electroencephalogram is sensed by the electrode unit (11) in an acquisition mode. Therefore, if the electroencephalogram is sensed by the electrode unit (11) in bad condition, then the condition of contact between the region of interest (51), forming part of the subject's (5) head (52), and the electrode unit (11), may be rectified, thus improving the measurement accuracy of the electroencephalogram information.

An electroencephalogram measurement system (10) according to a second aspect, which may be implemented in conjunction with the first aspect, further includes an electroencephalogram information presentation unit (213) to present the electroencephalogram information acquired by the acquisition unit (211).

This aspect allows the user (who may be either the subject (5) or medical staff) to learn the subject's (5) electroencephalogram based on the electroencephalogram information presented by the electroencephalogram information presentation unit (213).

An electroencephalogram measurement system (10) according to a third aspect, which may be implemented in conjunction with the first or second aspect, further includes a decision unit (214) to determine, based on the impedance value measured, whether the electroencephalogram is sensed in good condition or not.

This aspect allows the user (who may be either the subject (5) or medical staff) to learn, based on the decision made by the decision unit (214), a specific condition in which the electroencephalogram is sensed by the electrode unit (11).

In an electroencephalogram measurement system (10) according to a fourth aspect, which may be implemented in conjunction with the third aspect, the electrode unit (11) includes a plurality of electrode units (11). The impedance measurement unit (121) measures the impedance value between each of the plurality of electrode units (11) and the subject's (5) body. The decision unit (214) further determines, based on a relative relation in the impedance value between the plurality of electrode units (11), whether the electroencephalogram is sensed by the plurality of electrode units (11) in good condition or not.

This aspect allows the decision unit (214) to determine, with a relative relation in impedance value between a plurality of electrode units (11) taken into consideration, whether the electroencephalogram is sensed by the plurality of electrode units (11) in good condition or not. For example, when detecting that the difference in impedance value between the plurality of electrode units (11) has increased, the decision unit (214) may determine that the electroencephalogram should be sensed in bad condition.

An electroencephalogram measurement system (10) according to a fifth aspect, which may be implemented in conjunction with the third or fourth aspect, further includes a decision presentation unit (215) to present a decision made by the decision unit (214).

This aspect allows the user (who may be either the subject (5) or medical staff) to learn, based on the decision made by the decision unit (214) and presented to him or her by the decision presentation unit (215), whether the electroencephalogram is sensed by the electrode unit (11) in good condition or not.

In an electroencephalogram measurement system (10) according to a sixth aspect, which may be implemented in conjunction with the fifth aspect, the decision presentation unit (215) indicates, in colors, whether the electroencephalogram is sensed in good condition or not according to the decision made by the decision unit (214).

This aspect allows the user (who may be either the subject (5) or medical staff) to learn, by colors, whether the electroencephalogram is sensed in good condition or not.

In an electroencephalogram measurement system (10) according to a seventh aspect, which may be implemented in conjunction with the fifth or sixth aspect, the decision presentation unit (215) indicates a position of the electrode unit (11) using a figure representing the head (52).

This aspect allows the user (who may be either the subject (5) or medical staff) to visually learn the position of the electrode unit (11) on the head (52).

In an electroencephalogram measurement system (10) according to an eighth aspect, which may be implemented in conjunction with any one of the first to seventh aspects, measuring and recording the electroencephalogram using the electrode unit (11) and measuring the impedance value using the impedance measurement unit (121) are performed in parallel with each other.

This aspect allows the user (who may be either the subject (5) or medical staff) to learn the impedance value between the electrode unit (11) and the subject's (5) body when an electroencephalogram is obtained by the electrode unit (11).

In an electroencephalogram measurement system (10) according to a ninth aspect, which may be implemented in conjunction with any one of the first to eighth aspects, the output unit (212) displays, on a display unit (26), the impedance value measured as a numerical value, of which the number of significant digits corresponds to setting information.

This aspect allows the user to learn, based on the numerical value of the impedance value displayed on the display unit (26), the condition of contact between the electrode unit (11) and the subject's (5) body.

A rehabilitation support system (100) according to a tenth aspect includes the electroencephalogram measurement system (10) according to any one of the first to ninth aspects, an exercise assisting device (3), and a controller (4). The exercise assisting device (3) applies at least one of a mechanical stimulus or an electrical stimulus to the subject (5). The controller (4) controls the exercise assisting device (3) in accordance with the electroencephalogram information acquired by the acquisition unit (211).

This aspect allows the user to learn, based on the impedance value measured as provided by the output unit (212), a specific condition in which an electroencephalogram is sensed by the electrode unit (11) in an acquisition mode. Therefore, if the electroencephalogram is sensed by the electrode unit (11) in bad condition, then the condition of contact between the region of interest (51), forming part of the subject's (5) head (52), and the electrode unit (11), may be rectified, thus improving the measurement accuracy of the electroencephalogram information.

A method for controlling an electroencephalogram measurement system according to an eleventh aspect includes acquiring electroencephalogram information representing an electroencephalogram obtained by an electrode unit (11) that is placed on a region of interest (51) forming part of a subject's (5) head (52). The control method further includes measuring an impedance value between the electrode unit (11) and the subject's (5) body in an acquisition mode in which the electroencephalogram information is acquired; and outputting the impedance value measured.

This aspect allows the user to learn, based on the impedance value measured, a specific condition in which an electroencephalogram is sensed by the electrode unit (11) in an acquisition mode. Therefore, if the electroencephalogram is sensed by the electrode unit (11) in bad condition, then the condition of contact between the region of interest (51), forming part of the subject's (5) head (52), and the electrode unit (11), may be rectified, thus improving the measurement accuracy of the electroencephalogram information.

A program according to a twelfth aspect is designed to cause a computer system to carry out the method for controlling an electroencephalogram measurement system according to the eleventh aspect.

This aspect allows the user to learn, based on the impedance value measured, a specific condition in which an electroencephalogram is sensed by the electrode unit (11) in an acquisition mode. Therefore, if the electroencephalogram is sensed by the electrode unit (11) in bad condition, then the condition of contact between the region of interest (51), forming part of the subject's (5) head (52), and the electrode unit (11), may be rectified, thus improving the measurement accuracy of the electroencephalogram information.

A non-transitory storage medium according to a thirteenth aspect is readable for a computer system and stores a program designed to cause the computer system to carry out the method for controlling an electroencephalogram measurement system according to the eleventh aspect.

This aspect allows the user to learn, based on the impedance value measured, a specific condition in which an electroencephalogram is sensed by the electrode unit (11) in an acquisition mode. Therefore, if the electroencephalogram is sensed by the electrode unit (11) in bad condition, then the condition of contact between the region of interest (51), forming part of the subject's (5) head (52), and the electrode unit (11), may be rectified, thus improving the measurement accuracy of the electroencephalogram information.

Note that these aspects are only exemplary aspects of the present disclosure. Various configurations (including their variations) for the electroencephalogram measurement system (10) according to the embodiment described above may also be implemented as a method for controlling an electroencephalogram measurement system, a (computer) program, or a non-transitory storage medium on which the program is stored.

Also, the constituent elements according to the second to tenth aspects are not essential constituent elements for the rehabilitation support system (100) but may be omitted as appropriate.

### Reference Signs List

- 3: Exercise Assisting Device
- 4: Controller
- 5: Subject
- 10: Electroencephalogram Measurement System
- 11: Electrode Unit
- 51: Region of Interest
- 52: Head
- 100: Rehabilitation Support System
- 111: First Electrode (Electrode Unit)
- 112: Second Electrode (Electrode Unit)
- 121: Impedance Measurement Unit
- 211: Acquisition Unit
- 212: Output Unit
- 213: Electroencephalogram Information Presentation Unit
- 214: Decision Unit
- 215: Decision Presentation Unit
- 511: First Region of Interest (Region of Interest)
- 512: Second Region of Interest (Region of Interest)

## Claims

1. A rehabilitation support system (100) comprising an electroencephalogram measurement system (10), the electroencephalogram measurement system (10) comprising:
an acquisition unit (211) configured to acquire electroencephalogram information, the electroencephalogram information representing an electroencephalogram obtained by an electrode unit (11), the electrode unit (11) being placed on a region of interest forming part of a subject's (5) head (52);
an impedance measurement unit (121) configured to measure an impedance value between the electrode unit (11) and the subject's (5) body in an acquisition mode in which the acquisition unit (211) acquires the electroencephalogram information; and
an output unit (212) configured to output the impedance value measured,
wherein the rehabilitation support system (100) further comprises
an exercise assisting device (3) configured to apply at least one of a mechanical stimulus or an electrical stimulus to the subject (5);
a controller (4) configured to control the exercise assisting device (3) in accordance with the electroencephalogram information acquired by the acquisition unit (211); and
a headset (1), wherein the headset (1) includes the electrode unit (11), a signal processing unit (12) and a communications unit (13), wherein the signal processing unit (12) is configured to perform the function as the impedance measurement unit (121) and the communications unit (13) has the capability of communicating with an information processor (2) including a processor (21) having functions serving as the acquisition unit (211) and the output unit (212),
wherein the electrode unit (11) includes a first electrode (111) and a second electrode (112),
wherein the headset (1) further includes a reference electrode (113) and a ground electrode (114),
wherein the headset (1) further (1) includes an AC current source (14) for measuring the impedance value between the electrode unit (11) and the subject's (5) body,
wherein the AC current source (14) allows an alternating current to flow between the first electrode (111) and the reference electrode (113), and between the second electrode (112) and the reference electrode (113),
wherein the impedance measurement unit (121) is configured to detect a value of a voltage generated between the first electrode (111) and the reference electrode (113) and a value of a voltage between the second electrode (112) and the reference electrode (113),
wherein the impedance measurement unit (121) is configured to measure, based on the value of the voltage between the first electrode (111) and the reference electrode (113), an impedance value between the first electrode (111) and the body,
wherein the impedance measurement unit (121) is configured to measure, based on the value of the voltage between the second electrode (112) and the reference electrode (113), an impedance value between the second electrode (112) and the body, wherein the AC current source (14) is configured to supply an alternating current falling within a frequency band from a few hundred Hz to several kHz.

2. The rehabilitation support system (100) of claim 1, wherein the electroencephalogram measurement system (10) further comprises an electroencephalogram information presentation unit (213) configured to present the electroencephalogram information acquired by the acquisition unit (211).

3. The rehabilitation support system (100) of claim 1 or 2, wherein the electroencephalogram measurement system (10) further comprises a decision unit (214) configured to determine, based on the impedance value measured, whether the electroencephalogram is sensed by the electrode unit (11) in good condition or not.

4. The rehabilitation support system (100) of claim 3, wherein
the electrode unit (11) includes a plurality of electrode units,
the impedance measurement unit is configured to measure the impedance value between each of the plurality of electrode units and the subject's (5) body, and
the decision unit (214) is configured to further determine, based on a relative relation in the impedance value between the plurality of electrode units, whether the electroencephalogram is sensed by the plurality of electrode units in good condition or not.

5. The rehabilitation support system (100) of claim 3 or 4, wherein the electroencephalogram measurement system (10) further comprises a decision presentation unit (215) configured to present a decision made by the decision unit (214).

6. The rehabilitation support system (100) of claim 5, wherein
the decision presentation unit (215) is configured to indicate, in colors, whether the electroencephalogram is sensed in good condition or not according to the decision made by the decision unit (214).

7. The rehabilitation support system (100) of claim 5 or 6, wherein
the decision presentation unit (215) is configured to indicate a position of the electrode unit (11) using a figure representing the head (52).3 to 7

8. The rehabilitation support system (100) of any one of claims 1 to 7, wherein
measuring and recording the electroencephalogram using the electrode unit (11) and measuring the impedance value using the impedance measurement unit (121) are performed in parallel with each other.

9. The rehabilitation support system (100) of any one of claims 1 to 8, wherein
the output unit (212) is configured to display, on a display unit (26), the impedance value measured as a numerical value, of which the number of significant digits corresponds to setting information.

10. The rehabilitation support system (100) of any one of claims 1 to 9, wherein
a figure (M10) representing the head (52) is displayed on a display unit (26), and a mark representing the electrode unit (11) is displayed at a location corresponding to the electrode unit in the figure (M10),
wherein a display area (G91) is provided over the mark (M11) and the impedance value is shown in the display area (G91).

11. A method for controlling the rehabilitation support system (100) according to any one of claims 1 to 10, the method comprising:
acquiring electroencephalogram information representing an electroencephalogram obtained by the electrode unit (11), the electrode unit (11) being placed on a region of interest forming part of a subject's (5) head (52);
measuring an impedance value between the electrode unit (11) and the subject's (5) body in an acquisition mode in which the electroencephalogram information is acquired; and
outputting the impedance value measured-,
applying at least one of a mechanical stimulus or an electrical stimulus to the subject (5); and
controlling the exercise assisting device (3) in accordance with the electroencephalogram information,
wherein the AC current source (14) allows an alternating current to flow between the first electrode (111) and the reference electrode (113), and between the second electrode (112) and the reference electrode (113),
wherein the method further includes
detecting a value of a voltage generated between the first electrode (111) and the reference electrode (113) and a value of a voltage between the second electrode (112) and the reference electrode (113),
measuring, based on the value of the voltage between the first electrode (111) and the reference electrode (113), an impedance value between the first electrode (111) and the body,
measuring, based on the value of the voltage between the second electrode (112) and the reference electrode (113), an impedance value between the second electrode (112) and the body,
wherein the AC current source (14) supplies an alternating current falling within a frequency band from a few hundred Hz to several kHz.

## Patentansprüche

1. Rehabilitationsunterstützungssystem (100), umfassend ein Elektroenzephalogramm-Messsystem (10), wobei das Elektroenzephalogramm-Messsystem (10) Folgendes umfasst:
eine Erfassungseinheit (211), die so konfiguriert ist, dass sie Elektroenzephalogramm-Informationen erfasst, wobei die Elektroenzephalogramm-Informationen ein Elektroenzephalogramm darstellen, das von einer Elektrodeneinheit (11) erhalten wird, wobei die Elektrodeneinheit (11) auf einem Untersuchungsbereich platziert ist, der einen Teil des Kopfes (52) einer Person (5) ausbildet;
eine Impedanzmesseinheit (121), die so konfiguriert ist, dass sie einen Impedanzwert zwischen der Elektrodeneinheit (11) und dem Körper der Person (5) in einem Erfassungsmodus misst, in dem die Erfassungseinheit (211) die Elektroenzephalogramm-Informationen erfasst; und
eine Ausgabeeinheit (212), die so konfigurierbar ist, dass sie den gemessenen Impedanzwert ausgibt,
wobei das Rehabilitationsunterstützungssystem (100) ferner Folgendes umfasst
eine Übungshilfevorrichtung (3), die so konfiguriert ist, dass sie mindestens einen mechanischen oder einen elektrischen Reiz auf die Person (5) ausübt;
eine Steuereinheit (4), die so konfiguriert ist, dass sie die Übungshilfevorrichtung (3) in Übereinstimmung mit den Elektroenzephalogramm-Informationen steuert, die von der Erfassungseinheit (211) erfasst werden; und
ein Headset (1), wobei das Headset (1) die Elektrodeneinheit (11), eine Signalverarbeitungseinheit (12) und eine Kommunikationseinheit (13) beinhaltet, wobei die Signalverarbeitungseinheit (12) so konfiguriert ist, dass sie die Funktion als Impedanzmesseinheit (121) durchführt, und die Kommunikationseinheit (13) in der Lage ist, mit einem Informationsprozessor (2) zu kommunizieren, der einen Prozessor (21) mit Funktionen beinhaltet, die als die Erfassungseinheit (211) und die Ausgabeeinheit (212) dienen,
wobei die Elektrodeneinheit (11) eine erste Elektrode (111) und eine zweite Elektrode (112) beinhaltet,
wobei das Headset (1) ferner eine Referenzelektrode (113) und eine Masseelektrode (114) beinhaltet,
wobei das Headset (1) ferner (1) eine Wechselstromquelle (14) zum Messen des Impedanzwertes zwischen der Elektrodeneinheit (11) und dem Körper der Testperson (5) beinhaltet,
wobei die Wechselstromquelle (14) einen Wechselstrom zwischen der ersten Elektrode (111) und der Referenzelektrode (113) und zwischen der zweiten Elektrode (112) und der Referenzelektrode (113) strömen lässt,
wobei die Impedanzmesseinheit (121) so konfiguriert ist, dass sie einen Wert einer Spannung, die zwischen der ersten Elektrode (111) und der Referenzelektrode (113) erzeugt wird, und einen Wert einer Spannung zwischen der zweiten Elektrode (112) und der Referenzelektrode (113) erkennt,
wobei die Impedanzmesseinheit (121) so konfiguriert ist, dass sie, anhand des Werts der Spannung zwischen der ersten Elektrode (111) und der Referenzelektrode (113), einen Impedanzwert zwischen der ersten Elektrode (111) und dem Körper misst,
wobei die Impedanzmesseinheit (121) so konfiguriert ist, dass sie anhand des Werts der Spannung zwischen der zweiten Elektrode (112) und der Referenzelektrode (113) einen Impedanzwert zwischen der zweiten Elektrode (112) und dem Körper misst, wobei die Wechselstromquelle (14) so konfiguriert ist, dass sie einen Wechselstrom zuführt, der innerhalb eines Frequenzbands von einigen hundert Hz bis zu mehreren kHz fällt.

2. Rehabilitationsunterstützungssystem (100) nach Anspruch 1, wobei das Elektroenzephalogramm-Messsystem (10) ferner eine Elektroenzephalogramm-Informationsdarstellungseinheit (213) umfasst, die so konfiguriert ist, dass sie die Elektroenzephalogramm-Information darstellt, die von der Erfassungseinheit (211) erfasst wird.

3. Rehabilitationsunterstützungssystem (100) nach Anspruch 1 oder 2, wobei das Elektroenzephalogramm-Messsystem (10) ferner eine Entscheidungseinheit (214) umfasst, die so konfiguriert ist, dass sie anhand des gemessenen Impedanzwerts bestimmt, ob das Elektroenzephalogramm von der Elektrodeneinheit (11) in gutem Zustand abgetastet wird oder nicht.

4. Rehabilitationsunterstützungssystem (100) nach Anspruch 3, wobei die Elektrodeneinheit (11) eine Vielzahl von Elektrodeneinheiten beinhaltet,
die Impedanzmesseinheit so konfiguriert ist, dass sie den Impedanzwert zwischen jeder der Vielzahl von Elektrodeneinheiten und dem Körper des Subjekts (5) misst, und
die Entscheidungseinheit (214) so konfiguriert ist, dass sie ferner anhand eines relativen Verhältnisses in dem Impedanzwert zwischen der Vielzahl von Elektrodeneinheiten bestimmt, ob das Elektroenzephalogramm von der Vielzahl von Elektrodeneinheiten in gutem Zustand abgetastet wird oder nicht.

5. Rehabilitationsunterstützungssystem (100) nach Anspruch 3 oder 4, wobei das Elektroenzephalogramm-Messsystem (10) ferner eine Entscheidungsdarstellungseinheit (215) umfasst, die so konfiguriert ist, dass sie eine Entscheidung darstellt, die von der Entscheidungseinheit (214) getroffen wird.

6. Rehabilitationsunterstützungssystem (100) nach Anspruch 5, wobei
die Entscheidungsdarstellungseinheit (215) so konfiguriert ist, dass sie in Farben anzeigt, ob das Elektroenzephalogramm gemäß der Entscheidung, die von der Entscheidungseinheit (214) getroffen wird, in gutem Zustand abgetastet wird oder nicht.

7. Rehabilitationsunterstützungssystem (100) nach Anspruch 5 oder 6, wobei
die Entscheidungsdarstellungseinheit (215) so konfiguriert ist, dass sie eine Position der Elektrodeneinheit (11) unter Verwendung einer Figur anzeigt, die den Kopf (52) darstellt.

8. Rehabilitationsunterstützungssystem (100) nach einem der Ansprüche 1 bis 7, wobei
das Messen und Aufzeichnen des Elektroenzephalogramms mit der Elektrodeneinheit (11) und das Messen des Impedanzwertes mit der Impedanzmesseinheit (121) parallel zueinander durchgeführt werden.

9. Rehabilitationsunterstützungssystem (100) nach einem der Ansprüche 1 bis 8, wobei
die Ausgabeeinheit (212) so konfiguriert ist, dass sie auf einer Anzeigeeinheit (26) den gemessenen Impedanzwert als Zahlenwert anzeigt, dessen Anzahl signifikanter Ziffern einer Einstellungsinformation entspricht.

10. Rehabilitationsunterstützungssystem (100) nach einem der Ansprüche 1 bis 9, wobei
eine Figur (M10), die den Kopf (52) darstellt, auf einer Anzeigeeinheit (26) angezeigt wird, und eine Markierung, die die Elektrodeneinheit (11) darstellt, an einer Stelle angezeigt wird, die der Elektrodeneinheit in der Figur (M10) entspricht,
wobei ein Anzeigebereich (G91) über der Markierung (M11) bereitgestellt ist und der Impedanzwert in dem Anzeigebereich (G91) gezeigt wird.

11. Verfahren zum Steuern des Rehabilitationsunterstützungssystems (100) nach einem der Ansprüche 1 bis 10, wobei das Verfahren Folgendes umfasst:
Erfassen von Elektroenzephalogramm-Informationen, wobei die Elektroenzephalogramm-Informationen ein Elektroenzephalogramm darstellen, das von einer Elektrodeneinheit (11) erhalten wird, wobei die Elektrodeneinheit (11) auf einem Untersuchungsbereich platziert ist, der einen Teil des Kopfes (52) einer Person (5) ausbildet;
Messen eines Impedanzwertes zwischen der Elektrodeneinheit (11) und dem Körper der Person (5) in einem Erfassungsmodus, in dem die Elektroenzephalogramm-Informationen erfasst werden; und
Ausgeben des gemessenen Impedanzwerts,
Anwenden mindestens eines mechanischen oder eines elektrischen Reizes auf die Person (5);
Steuern der Übungshilfevorrichtung (3) in Übereinstimmung mit den Elektroenzephalogramm-Informationen,
wobei die Wechselstromquelle (14) einen Wechselstrom zwischen der ersten Elektrode (111) und der Referenzelektrode (113) und zwischen der zweiten Elektrode (112) und der Referenzelektrode (113) strömen lässt,
wobei das Verfahren ferner Folgendes beinhaltet
Erkennen eines Werts einer Spannung, die zwischen der ersten Elektrode (111) und der Referenzelektrode (113) erzeugt wird, und eines Werts einer Spannung zwischen der zweiten Elektrode (112) und der Referenzelektrode (113),
Messen, anhand des Werts der Spannung zwischen der ersten Elektrode (111) und der Referenzelektrode (113), eines Impedanzwerts zwischen der ersten Elektrode (111) und dem Körper,
Messen, anhand des Werts der Spannung zwischen der zweiten Elektrode (112) und der Referenzelektrode (113), eines Impedanzwerts zwischen der zweiten Elektrode (112) und dem Körper,
wobei die Wechselstromquelle (14) einen Wechselstrom zuführt, der innerhalb eines Frequenzbands von einigen hundert Hz bis zu mehreren kHz fällt.

## Revendications

1. Système de support de rééducation (100) comprenant un système de mesure d'électroencéphalogramme (10), le système de mesure d'électroencéphalogramme (10) comprenant :
une unité d'acquisition (211) configurée pour acquérir des informations d'électroencéphalogramme, les informations d'électroencéphalogramme représentant un électroencéphalogramme obtenu par une unité d'électrodes (11), l'unité d'électrodes (11) étant placée sur une région d'intérêt faisant partie de la tête (52) d'un sujet (5) ;
une unité de mesure d'impédance (121) configurée pour mesurer une valeur d'impédance entre l'unité d'électrodes (11) et le corps du sujet (5) dans un mode d'acquisition dans lequel l'unité d'acquisition (211) acquiert les informations d'électroencéphalogramme ; et
une unité de sortie (212) configurée pour fournir en sortie la valeur d'impédance mesurée, dans lequel le système de support de rééducation (100) comprend en outre
un dispositif d'assistance à l'exercice (3) configuré pour appliquer au moins l'un parmi un stimulus mécanique ou un stimulus électrique au sujet (5) ;
un contrôleur (4) configuré pour commander le dispositif d'assistance à l'exercice (3) conformément aux informations d'électroencéphalogramme acquises par l'unité d'acquisition (211) ; et
un casque (1), dans lequel le casque (1) comprend l'unité d'électrodes (11), une unité de traitement de signal (12) et une unité de communication (13), dans laquelle l'unité de traitement de signal (12) est configurée pour remplir la fonction d'unité de mesure d'impédance (121), et l'unité de communication (13) a la capacité de communiquer avec un processeur d'informations (2) comprenant un processeur (21) ayant des fonctions servant d'unité d'acquisition (211) et d'unité de sortie (212),
dans laquelle l'unité d'électrodes (11) comprend une première électrode (111) et une deuxième électrode (112),
dans lequel le casque (1) comprend en outre une électrode de référence (113) et une électrode de masse (114),
dans lequel le casque (1) comprend en outre (1) une source de courant alternatif (14) pour mesurer la valeur d'impédance entre l'unité d'électrodes (11) et le corps du sujet (5),
dans laquelle la source de courant alternatif (14) permet à un courant alternatif de circuler entre la première électrode (111) et l'électrode de référence (113), et entre la deuxième électrode (112) et l'électrode de référence (113),
dans laquelle l'unité de mesure d'impédance (121) est configurée pour détecter une valeur d'une tension générée entre la première électrode (111) et l'électrode de référence (113) et une valeur d'une tension entre la deuxième électrode (112) et l'électrode de référence (113),
dans laquelle l'unité de mesure d'impédance (121) est configurée pour mesurer, sur la base de la valeur de la tension entre la première électrode (111) et l'électrode de référence (113), une valeur d'impédance entre la première électrode (111) et le corps,
dans laquelle l'unité de mesure d'impédance (121) est configurée pour mesurer, sur la base de la valeur de la tension entre la deuxième électrode (112) et l'électrode de référence (113), une valeur d'impédance entre la deuxième électrode (112) et le corps, dans laquelle la source de courant alternatif (14) est configurée pour fournir un courant alternatif situé dans une bande de fréquences allant de quelques centaines de Hz à plusieurs kHz.

2. Système de support de rééducation (100) selon la revendication 1, dans lequel le système de mesure d'électroencéphalogramme (10) comprend en outre une unité de présentation d'informations d'électroencéphalogramme (213) configurée pour présenter les informations d'électroencéphalogramme acquises par l'unité d'acquisition (211).

3. Système de support de rééducation (100) selon la revendication 1 ou 2, dans lequel le système de mesure d'électroencéphalogramme (10) comprend en outre une unité de décision (214) configurée pour déterminer, sur la base de la valeur d'impédance mesurée, si l'électroencéphalogramme est détecté par l'unité d'électrodes (11) dans de bonnes conditions ou non.

4. Système de support de rééducation (100) selon la revendication 3, dans lequel
l'unité d'électrodes (11) comprend une pluralité d'unités d'électrodes,
l'unité de mesure d'impédance est configurée pour mesurer la valeur d'impédance entre chacune des unités d'électrodes et le corps du sujet (5), et
l'unité de décision (214) est configurée pour déterminer en outre, sur la base d'une relation relative dans la valeur d'impédance entre les unités d'électrodes, si l'électroencéphalogramme est détecté par les unités d'électrodes dans de bonnes conditions ou non.

5. Système de support de rééducation (100) selon la revendication 3 ou 4, dans lequel le système de mesure d'électroencéphalogramme (10) comprend en outre une unité de présentation de décision (215) configurée pour présenter une décision prise par l'unité de décision (214).

6. Système de support de rééducation (100) selon la revendication 5, dans lequel
l'unité de présentation de décision (215) est configurée pour indiquer, à l'aide de couleurs, si l'électroencéphalogramme est détecté dans de bonnes conditions ou non, conformément à la décision prise par l'unité de décision (214).

7. Système de support de rééducation (100) selon la revendication 5 ou 6, dans lequel
l'unité de présentation de décision (215) est configurée pour indiquer une position de l'unité d'électrodes (11) à l'aide d'une des figures 3 à 7 représentant la tête (52).

8. Système de support de rééducation (100) selon l'une quelconque des revendications 1 à 7, dans lequel
les étapes de mesure et d'enregistrement de l'électroencéphalogramme à l'aide de l'unité d'électrodes (11) et de mesure de la valeur d'impédance à l'aide de l'unité de mesure d'impédance (121) sont effectuées en parallèle l'une avec l'autre.

9. Système de support de rééducation (100) selon l'une quelconque des revendications 1 à 8, dans lequel
l'unité de sortie (212) est configurée pour afficher, sur une unité d'affichage (26), la valeur d'impédance mesurée, sous la forme d'une valeur numérique, dont le nombre de chiffres significatifs correspond à des informations de réglage.

10. Système de support de rééducation (100) selon l'une quelconque des revendications 1 à 9, dans lequel
une figure (M10) représentant la tête (52) est affichée sur une unité d'affichage (26), et une marque représentant l'unité d'électrodes (11) est affichée à un emplacement correspondant à l'unité d'électrodes dans la figure (M10),
dans lequel une zone d'affichage (G91) est prévue au-dessus de la marque (M11) et la valeur d'impédance est affichée dans la zone d'affichage (G91).

11. Procédé de commande du système de support de rééducation (100) selon l'une quelconque des revendications 1 à 10, le procédé comprenant le fait de :
acquérir des informations d'électroencéphalogramme représentant un électroencéphalogramme obtenu par l'unité d'électrodes (11), l'unité d'électrodes (11) étant placée sur une région d'intérêt faisant partie de la tête (52) d'un sujet (5) ;
mesurer une valeur d'impédance entre l'unité d'électrodes (11) et le corps du sujet (5) dans un mode d'acquisition dans lequel les informations d'électroencéphalogramme sont acquises ; et
fournir en sortie la valeur d'impédance mesurée ;
appliquer au moins l'un parmi un stimulus mécanique ou un stimulus électrique au sujet (5) ;
commander le dispositif d'assistance à l'exercice (3) conformément aux informations d'électroencéphalogramme ;
dans laquelle la source de courant alternatif (14) permet à un courant alternatif de circuler entre la première électrode (111) et l'électrode de référence (113), et entre la deuxième électrode (112) et l'électrode de référence (113),
dans lequel le procédé comprend en outre le fait de :
détecter une valeur d'une tension générée entre la première électrode (111) et l'électrode de référence (113) et une valeur d'une tension entre la deuxième électrode (112) et l'électrode de référence (113),
mesurer, sur la base de la valeur de la tension entre la première électrode (111) et l'électrode de référence (113), une valeur d'impédance entre la première électrode (111) et le corps,
mesurer, sur la base de la valeur de la tension entre la deuxième électrode (112) et l'électrode de référence (113), une valeur d'impédance entre la deuxième électrode (112) et le corps,
dans laquelle la source de courant alternatif (14) fournit un courant alternatif situé dans une bande de fréquences allant de quelques centaines de Hz à plusieurs kHz.
